# EUROPEAN PATENT APPLICATION

(11) **EP 4 773 147 A2**
(43) Date of publication of application: **08.07.2026**
(21) Application number: 26165123.6
(22) Date of filing: 15.11.2020
(51) Int. Cl.: G16H 50/20

(54) **METHOD AND APPARATUS FOR VISUALIZATION OF BONE MARROW CELL POPULATIONS**

(30) Priority: 15.11.2019 US 201962935843 P; 30.06.2020 US 202062705501 P
(62) Divisional of application: 20888551.7
(71) Applicant: Scopio Labs Ltd., 6618502 Tel Aviv (IL)
(72) Inventor: Leshem, Ben, 6912207 Tel Aviv (IL); Small, Eran, 5631204 Yahud (IL); Naaman, Erez, 6522111 Tel Aviv (IL); Hayut, Itai, 6967162 Tel Aviv (IL); Karny, Shahar, 4528257 Hod Hasharon (IL); Aizenman, Ofek, 5849244 Holon (IL)
(74) Representative: Mathys & Squire

(57) **Abstract**

A microscope system for scanning a bone marrow aspirate (EMA) sample may include a scanning apparatus for scanning the EMA sam-ple and a processor coupled to the scanning apparatus and a memory. The mi-croscope system may obtain, using the scanning apparatus, scan data of the EMA sample and detect cells in the EMA sample from the scan data. The microscope system may also classify the detected cells into a plurality of cell types, and store, in the memory, cell data of the classified cells. The micro-scope system may include a display for presenting the cell data. Various other systems and methods are provided.

## Description

### RELATED APPLICATIONS

This application claims the benefit under 35 U.S.C. § 119(e) of U.S. Provisional Patent Application No. 62/935,843, filed November 15, 2019, and titled "VISUALIZATION OF BONE MARROW CELL POPULATIONS," and U.S. Provisional Patent Application No. 62/705,501, filed June 30, 2020, and titled "BONE MARROW ASPIRATE DIAGNOSTIC SYSTEM," both of which are incorporated, in their entirety, by this reference.

### BACKGROUND

Bone marrow aspirate analysis can be used in the diagnosis of many types of diseases, such as leukemia. However, work in relation to the present disclosure suggests that prior approaches to analyzing marrow samples can be less than ideal, and that improved analysis of bone marrow aspirate samples could help physicians make improved diagnoses of patients. The analysis of bone marrow aspirate ("BMA") samples can be a complicated and time-consuming process, and at least some of the data potentially available from BMA samples is underutilized, which can lead to an inaccurate analysis and diagnosis in at least some instances. For instance, after preparing a sample, an operator may analyze the sample under a microscope, in which at least part of the analysis is done using a high magnification with oil immersion, which has a small field of view. This approach can be time consuming and may lead to the operator not being able to review all of the potentially relevant cells within a reasonable time frame. Bone marrow sample analysis often relies on a differential analysis of the different cell types in the sample at different regions of the sample. The bone marrow sample analysis may involve analyzing the macro structure of the sample, often at a lower magnification, to check its adequacy and to find areas for analysis, and then looking, at a higher magnification, for relevant cells and performing a differential analysis on around 500 cells (according to ICSH guidelines). Work in relation to the present disclosure suggests that the sensitivity and specificity of prior BMA analysis can be less than ideal in at least some instances. For example with some diseases, the cells indicating the presence of a disease in the BMA sample may be present at a focal region of the BMA sample, which can be overlooked in at least some instances.

Traditionally the analysis is manually performed by a person. The person may review the sample under a microscope, choose a relevant area of the sample, identify various cell types in the relevant area, and based on the results (e.g., numbers of cells for each cell type) the person may attempt to diagnose the condition. Work in relation to the present disclosure suggests that prior approaches to determining the relevant area of the sample identification of the cell types can be somewhat subjective in at least some instances. With some prior approaches the relevant area of the sample may not be appropriately defined, and bias can be introduced into the analysis that can affect the accuracy of the analysis in at least some instances.

The prior approaches may suffer from additional limitations. For instance, because the analysis is performed manually, the person may rely on cognitive awareness to assess the clinical meaning of the different cell populations as well as a memory of the different conditions present. This may be exacerbated with many cells being difficult to differentiate as well as the challenge of seeking indicative cells that may be abnormal or otherwise indicate a certain medical condition. In addition, some diseases may appear in a specific part of the sample, which can be overlooked. The process may also be lengthy as the person may individually perform the analysis and repeat the entire process for multiple slides. As such, the traditional approach may be prone to human error in at least some instances. Also, the prior approaches have been somewhat limited because the types of analysis performed have been somewhat limited by the cognitive abilities of the individual performing the analysis, and work in relation to the present disclosure suggests that relationships among cell types and lineages may not have been appropriately utilized in at least some instances. Moreover, because many healthcare facilities lack someone with a requisite level of expertise, at least some facilities may rely upon remote experts by physically shipping the sample, further adding to the overhead and delays for analyzing the sample.

### SUMMARY

The systems and methods described herein may provide a computer-assisted system for bone marrow aspirate analysis, including a visualization of bone marrow cell populations, which can be used by a care provider to perform a diagnosis. In some embodiments, the presently disclosed systems and methods allow quantitative analysis of a large number of cells over a large area, which can increase the sensitivity and specificity of the BMA analysis. Also, the areas for analysis can be selected in ways that decrease bias in the analysis. While the areas for analysis can be selected in many ways, in some embodiments areas for analysis are selected that are representative of other areas. Alternatively or in combination, cells from other areas that are representative of cells in a given area are chosen for analysis, which can improve the reliability of the areas chosen for analysis and decrease bias.

In some embodiments, scan data from the BMA sample is received. One or more of particles or cell locations is detected from the scan data, and an analysis area determined based on the detected one or more of particles or cell locations. Cells of one or more cell types can be identified from the scan data. The identified cells can be analyzed with statistical analysis, and a subset of the identified cells selected for presentation based on the statistical analysis, which can facilitate a diagnosis by a health care provider.

In some embodiments, the region of interest determined quantitatively based on particles and cell densities of the sample, which can decrease bias in the BMA analysis. Also, representative cells within a region of interest can be selected. The different cell populations may be detected and presented in a way that may facilitate a diagnosis of a normal or abnormal cell population. The visual representation may use colors, sizes, shapes, text/numbers, and/or other visual indicators to present a cell lineage connection between types of cells, numbers or percentages of different cell types, an indication of amounts with respect to normal range values. The visual representation may advantageously facilitate quick diagnosis of abnormalities, communicate and trace decision making, enable comparison to an atlas of known conditions (e.g., graphs corresponding to known conditions) in order to reduce human error, improve the consistency and quality of diagnosis, and enable fast results.

### INCORPORATION BY REFERENCE

All patents, applications, and publications referred to and identified herein are hereby incorporated by reference in their entirety and shall be considered fully incorporated by reference even though referred to elsewhere in the application.

### BRIEF DESCRIPTION OF THE DRAWINGS

A better understanding of the features, advantages and principles of the present disclosure will be obtained by reference to the following detailed description that sets forth illustrative embodiments, and the accompanying drawings of which:
FIG. 1 shows a diagram of an exemplary microscope, in accordance with some embodiments;
FIG. 2 shows a chart of cell lineages, in accordance with some embodiments;
FIG. 3 shows another chart of cell lineages, in accordance with some embodiments;
FIG. 4 shows an exemplary cell linage analysis, in accordance with some embodiments;
FIG. 5 shows an exemplary scan analysis of a sample, in accordance with some embodiments;
FIG. 6 shows an exemplary heat map of cell density, in accordance with some embodiments;
FIG. 7 shows a flowchart of an example process for bone marrow scan analysis in classifying cells, in accordance with some embodiments;
FIG. 8 shows a flowchart of an example process for bone marrow scan analysis in selecting representative cells, in accordance with some embodiments;
FIG. 9 shows a flowchart of an example process for bone marrow scan analysis in cell population data, in accordance with some embodiments;
FIG. 10 shows a flowchart of an example process for bone marrow scan analysis in selecting representative cells, in accordance with some embodiments;
FIG. 11 shows a flowchart of an example process for reviewing a scan, in accordance with some embodiments;
FIG. 12 shows a flowchart of an example process for analyzing a scan, in accordance with some embodiments;
FIG. 13 shows a flowchart of an example process for bone marrow scan analysis, in accordance with some embodiments;
FIG. 14 shows an exemplary computing system, in accordance with some embodiments; and
FIG. 15 shows an exemplary network architecture, in accordance with some embodiments.

### DETAILED DESCRIPTION

The following detailed description and provides a better understanding of the features and advantages of the inventions described in the present disclosure in accordance with the embodiments disclosed herein. Although the detailed description includes many specific embodiments, these are provided by way of example only and should not be construed as limiting the scope of the inventions disclosed herein.

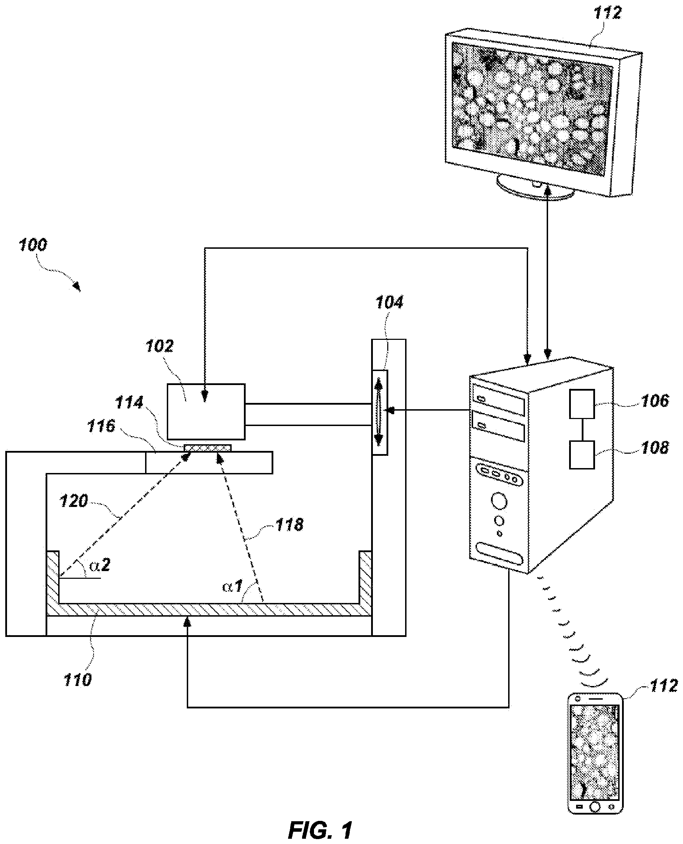

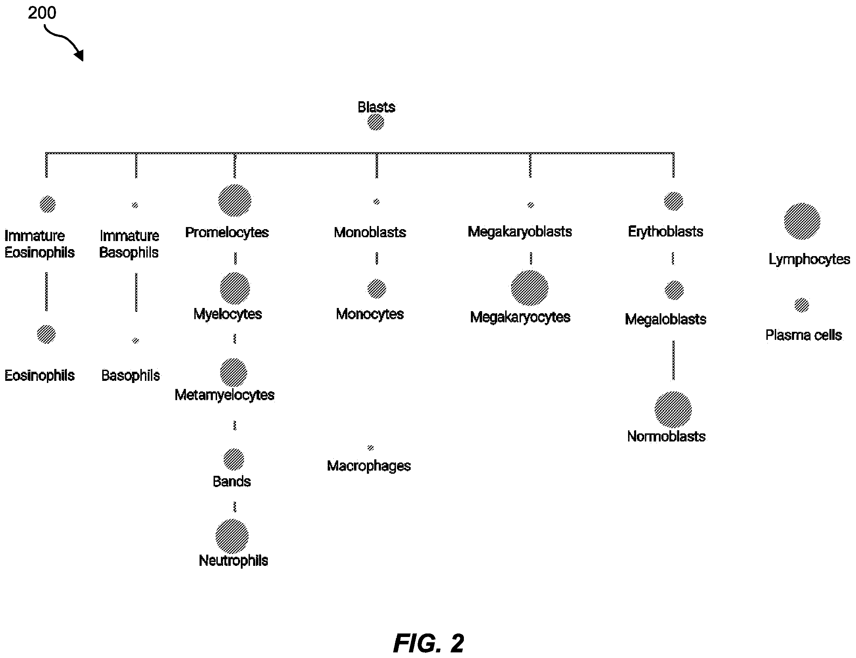

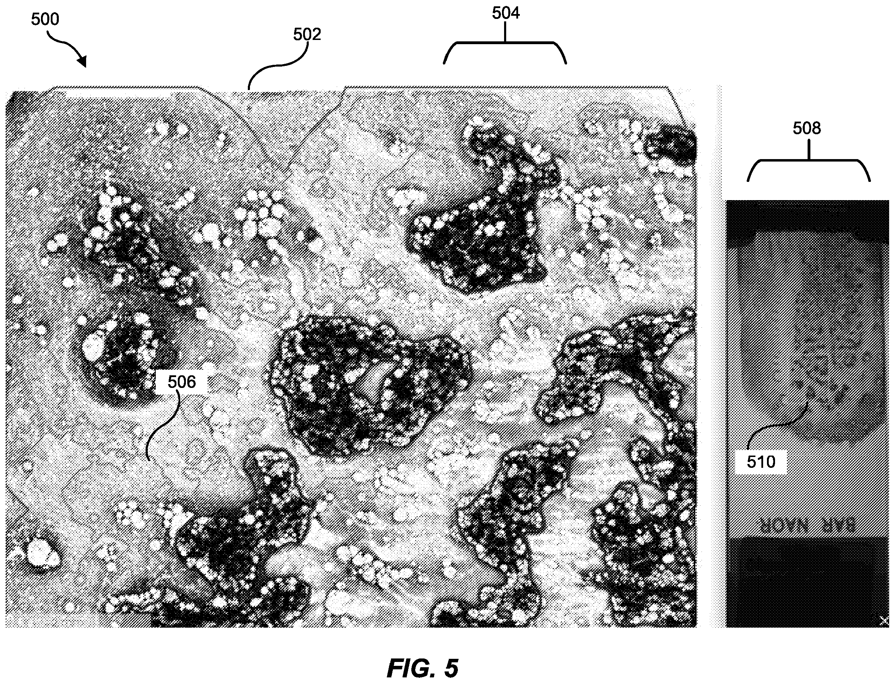

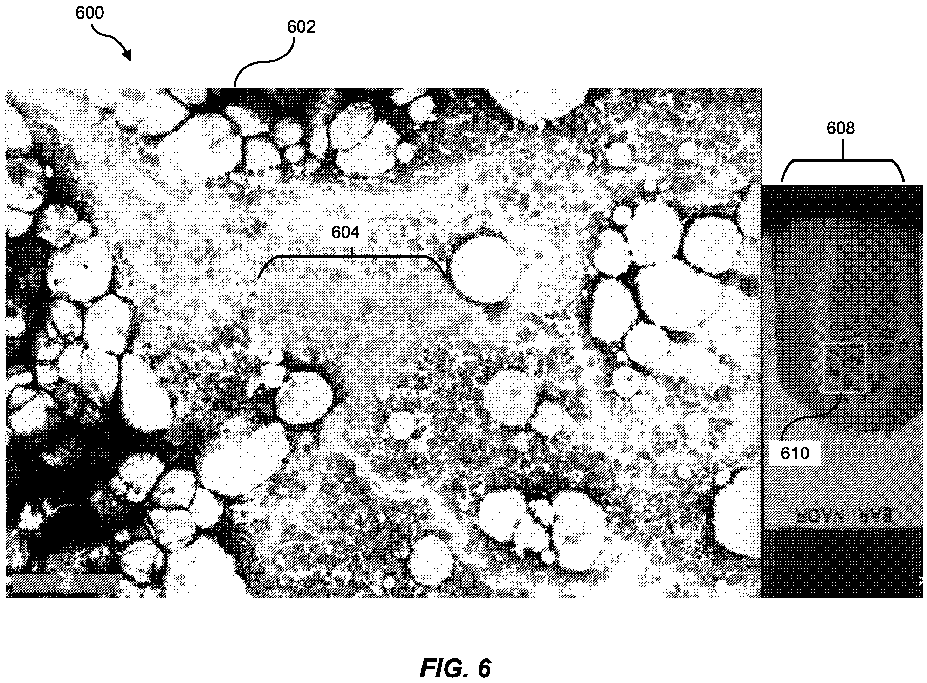

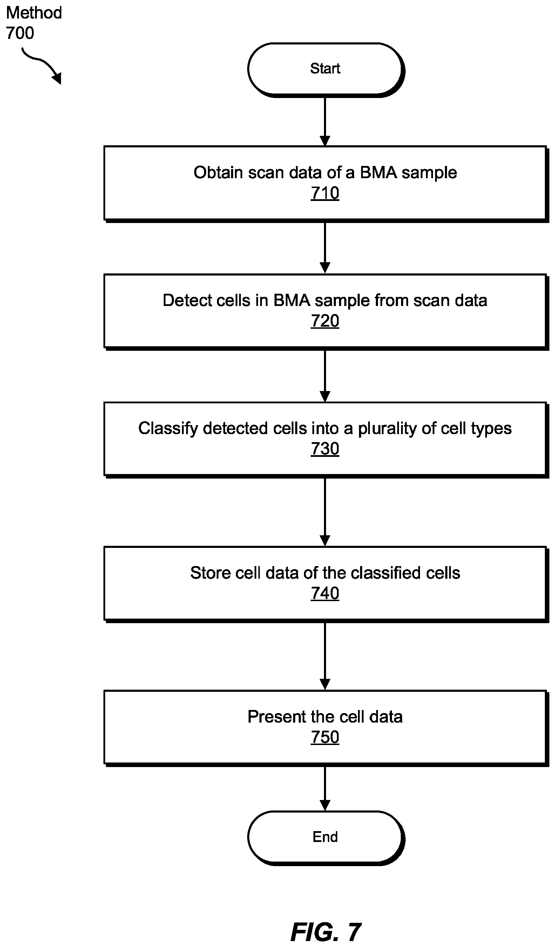

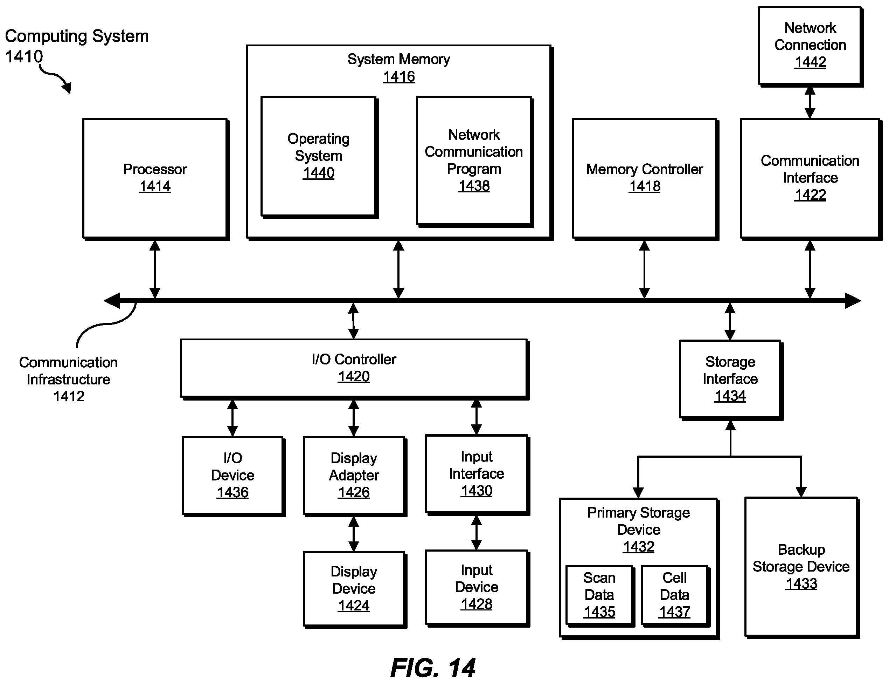

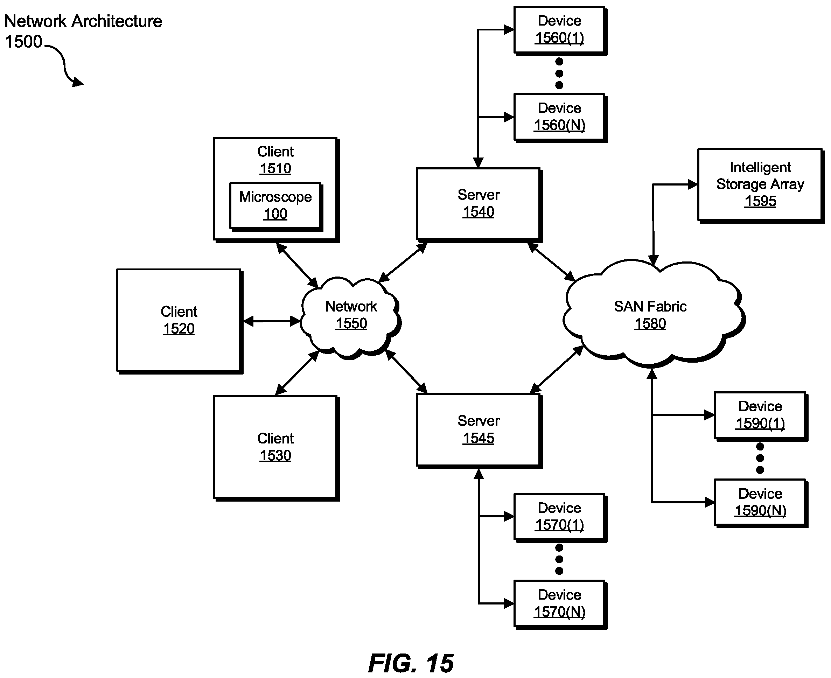

The following will provide, with reference to FIGS. 1-15, detailed descriptions of analysis of a BMA. FIG. 1 illustrates a microscope and various microscope configurations. FIGS. 2, 3, and 4 illustrate example charts of cell lineage analysis of a sample. FIGS. 5 and 6 illustrate example screenshots of scan results. FIGS. 7, 8, 9, 10, 11, 12, and 13 illustrate flow charts of example processes of bone marrow aspirate sample analysis. FIG. 14 illustrates an exemplary computing device capable of bone marrow aspirate sample analysis as described herein. FIG. 15 illustrates an exemplary networking architecture for computing devices described herein.

The presently disclosed systems and methods are well suited for use with prior methods of collecting bone marrow aspirate samples. The bone marrow aspirate sample can be obtained in the manner as would typically be done as will be appreciated by one of ordinary skill in the art. The bone marrow aspirate sample can be obtained from any species of human or animal that has bone marrow, and the sample can be obtained from a living subject such as a patient or a dead subject such as a cadaver.

FIG. 1 is a diagrammatic representation of a microscope 100 consistent with the exemplary disclosed embodiments. The term "microscope" as used herein generally refers to any device or instrument for magnifying an object which is smaller than easily observable by the naked eye, i.e., creating an image of an object for a user where the image is larger than the object. One type of microscope may be an "optical microscope" that uses light in combination with an optical system for magnifying an object. An optical microscope may be a simple microscope having one or more magnifying lens. Another type of microscope may be a "computational microscope" that comprises an image sensor and image-processing algorithms to enhance or magnify the object's size or other properties. The computational microscope may be a dedicated device or created by incorporating software and/or hardware with an existing optical microscope to produce high-resolution digital images. As shown in FIG. 1, microscope 100 comprises an image capture device 102, a focus actuator 104, a controller 106 connected to memory 108, an illumination assembly 110, and a user interface 112. An example usage of microscope 100 may be capturing images of a sample 114 mounted on a stage 116 located within the field-of-view (FOV) of image capture device 102, processing the captured images, and presenting on user interface 112 a magnified image of sample 114.

Image capture device 102 may be used to capture images of sample 114. In this specification, the term "image capture device" as used herein generally refers to a device that records the optical signals entering a lens as an image or a sequence of images. The optical signals may be in the near-infrared, infrared, visible, and ultraviolet spectrums. Examples of an image capture device comprise a CCD camera, a CMOS camera, a color camera, a photo sensor array, a video camera, a mobile phone equipped with a camera, a webcam, a preview camera, a microscope objective and detector, etc. Some embodiments may comprise only a single image capture device 102, while other embodiments may comprise two, three, or even four or more image capture devices 102. In some embodiments, image capture device 102 may be configured to capture images in a defined field-of-view (FOV). Also, when microscope 100 comprises several image capture devices 102, image capture devices 102 may have overlap areas in their respective FOVs. Image capture device 102 may have one or more image sensors (not shown in FIG. 1) for capturing image data of sample 114. In other embodiments, image capture device 102 may be configured to capture images at an image resolution higher than VGA, higher than 1 Megapixel, higher than 2 Megapixels, higher than 5 Megapixels, 10 Megapixels, higher than 12 Megapixels, higher than 15 Megapixels, or higher than 20 Megapixels. In addition, image capture device 102 may also be configured to have a pixel size smaller than 15 micrometers, smaller than 10 micrometers, smaller than 5 micrometers, smaller than 3 micrometers, or smaller than 1.6 micrometer.

In some embodiments, microscope 100 comprises focus actuator 104. The term "focus actuator" as used herein generally refers to any device capable of converting input signals into physical motion for adjusting the relative distance between sample 114 and image capture device 102. Various focus actuators may be used, including, for example, linear motors, electrostrictive actuators, electrostatic motors, capacitive motors, voice coil actuators, magnetostrictive actuators, etc. In some embodiments, focus actuator 104 may comprise an analog position feedback sensor and/or a digital position feedback element. Focus actuator 104 is configured to receive instructions from controller 106 in order to make light beams converge to form a clear and sharply defined image of sample 114. In the example illustrated in FIG. 1, focus actuator 104 may be configured to adjust the distance by moving image capture device 102.

However, in other embodiments, focus actuator 104 may be configured to adjust the distance by moving stage 116, or by moving both image capture device 102 and stage 116. Microscope 100 may also comprise controller 106 for controlling the operation of microscope 100 according to the disclosed embodiments. Controller 106 may comprise various types of devices for performing logic operations on one or more inputs of image data and other data according to stored or accessible software instructions providing desired functionality. For example, controller 106 may comprise a central processing unit (CPU), support circuits, digital signal processors, integrated circuits, cache memory, or any other types of devices for image processing and analysis such as graphic processing units (GPUs). The CPU may comprise any number of microcontrollers or microprocessors configured to process the imagery from the image sensors. For example, the CPU may comprise any type of single- or multi-core processor, mobile device microcontroller, etc. Various processors may be used, including, for example, processors available from manufacturers such as Intel^{®}, AMD^{®}, etc. and may comprise various architectures (e.g., x86 processor, ARM^{®}, etc.). The support circuits may be any number of circuits generally well known in the art, including cache, power supply, clock and input-output circuits. Controller 106 may be at a remote location, such as a computing device communicatively coupled to microscope 100.

In some embodiments, controller 106 may be associated with memory 108 used for storing software that, when executed by controller 106, controls the operation of microscope 100. In addition, memory 108 may also store electronic data associated with operation of microscope 100 such as, for example, captured or generated images of sample 114. In one instance, memory 108 may be integrated into the controller 106. In another instance, memory 108 may be separated from the controller 106.

Specifically, memory 108 may refer to multiple structures or computer-readable storage mediums located at controller 106 or at a remote location, such as a cloud server. Memory 108 may comprise any number of random access memories, read only memories, flash memories, disk drives, optical storage, tape storage, removable storage and other types of storage.

Microscope 100 may comprise illumination assembly 110. The term "illumination assembly" as used herein generally refers to any device or system capable of projecting light to illuminate sample 114.

Illumination assembly 110 may comprise any number of light sources, such as light emitting diodes (LEDs), LED array, lasers, and lamps configured to emit light, such as a halogen lamp, an incandescent lamp, or a sodium lamp. For example, illumination assembly 110 may comprise a Kohler illumination source. Illumination assembly 110 may be configured to emit polychromatic light. For instance, the polychromatic light may comprise white light.

In some embodiments, illumination assembly 110 may comprise only a single light source. Alternatively, illumination assembly 110 may comprise four, sixteen, or even more than a hundred light sources organized in an array or a matrix. In some embodiments, illumination assembly 110 may use one or more light sources located at a surface parallel to illuminate sample 114. In other embodiments, illumination assembly 110 may use one or more light sources located at a surface perpendicular or at an angle to sample 114.

In addition, illumination assembly 110 may be configured to illuminate sample 114 in a series of different illumination conditions. In one example, illumination assembly 110 may comprise a plurality of light sources arranged in different illumination angles, such as a two-dimensional arrangement of light sources. In this case, the different illumination conditions may comprise different illumination angles. For example, FIG. 1 depicts a beam 118 projected from a first illumination angle α1, and a beam 120 projected from a second illumination angle α2. In some embodiments, first illumination angle α1 and second illumination angle α2 may have the same value but opposite sign. In other embodiments, first illumination angle α1 may be separated from second illumination angle α2. However, both angles originate from points within the acceptance angle of the optics. In another example, illumination assembly 110 may comprise a plurality of light sources configured to emit light in different wavelengths. In this case, the different illumination conditions may comprise different wavelengths. For instance, each light source may be configured to emit light with a full width half maximum bandwidth of no more than 50 nm so as to emit substantially monochromatic light. In yet another example, illumination assembly 110 may be configured to use a number of light sources at predetermined times. In this case, the different illumination conditions may comprise different illumination patterns. For example, the light sources may be arranged to sequentially illuminate the sample at different angles to provide one or more of digital refocusing, aberration correction, or resolution enhancement. Accordingly and consistent with the present disclosure, the different illumination conditions may be selected from a group including: different durations, different intensities, different positions, different illumination angles, different illumination patterns, different wavelengths, or any combination thereof.

Although reference is made to computational microscopy, the presently disclosed systems and methods are well suited for use with many types of microscopy and microscopes such as one or more of a high definition microscope, a digital microscope, a scanning digital microscope, a 3D microscope, a phase imaging microscope, a phase contrast microscope, a dark field microscope, a differential interference contrast microscope, a light-sheet microscope, a confocal microscope, a holographic microscope, or a fluorescence-based microscope.

Consistent with disclosed embodiments, microscope 100 may comprise, be connected with, or in communication with (e.g., over a network or wirelessly, e.g., via Bluetooth) user interface 112. The term "user interface" as used herein generally refers to any device suitable for presenting a magnified image of sample 114 or any device suitable for receiving inputs from one or more users of microscope 100. FIG. 1 illustrates two examples of user interface 112. The first example is a smartphone or a tablet wirelessly communicating with controller 106 over a Bluetooth, cellular connection or a Wi-Fi connection, directly or through a remote server. The second example is a PC display physically connected to controller 106. In some embodiments, user interface 112 may comprise user output devices, including, for example, a display, tactile device, speaker, etc. In other embodiments, user interface 112 may comprise user input devices, including, for example, a touchscreen, microphone, keyboard, pointer devices, cameras, knobs, buttons, etc. With such input devices, a user may be able to provide information inputs or commands to microscope 100 by typing instructions or information, providing voice commands, selecting menu options on a screen using buttons, pointers, or eye-tracking capabilities, or through any other suitable techniques for communicating information to microscope 100. User interface 112 may be connected (physically or wirelessly) with one or more processing devices, such as controller 106, to provide and receive information to or from a user and process that information. In some embodiments, such processing devices may execute instructions for responding to keyboard entries or menu selections, recognizing and interpreting touches and/or gestures made on a touchscreen, recognizing and tracking eye movements, receiving and interpreting voice commands, etc.

Microscope 100 may also comprise or be connected to stage 116. Stage 116 comprises any horizontal rigid surface where sample 114 may be mounted for examination. Stage 116 may comprise a mechanical connector for retaining a slide containing sample 114 in a fixed position. The mechanical connector may use one or more of the following: a mount, an attaching member, a holding arm, a clamp, a clip, an adjustable frame, a locking mechanism, a spring or any combination thereof. In some embodiments, stage 116 may comprise a translucent portion or an opening for allowing light to illuminate sample 114. For example, light transmitted from illumination assembly 110 may pass through sample 114 and towards image capture device 102. In some embodiments, stage 116 and/or sample 114 may be moved using motors or manual controls in the XY plane to enable imaging of multiple areas of the sample.

FIG. 2 illustrates an exemplary chart 200 of a classification of cells in a sample that may correspond to a normal or nearly normal bone marrow sample. As will be described further below, microscope 100 may scan and further analyze a bone marrow aspirate sample. Some of the results of the analysis may be visually presented in a chart such as chart 200. For example, microscope 100 may classify the cells identified in the sample, which may include, for example, blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, plasma cells, etc.

Chart 200 may organize the cell types based on lineages. For example, as seen in FIG. 2, the different types of cells may be organized into columns by type with a maturation connection. In addition, each column may include a more granular cell progression (e.g., immature eosinophils to eosinophils, etc.). Certain cell types, such as lymphocytes, may be displayed separately, as seen in FIG. 2.

In addition, chart 200 may visually represent cell populations to reflect relative and/or absolute cell populations. In FIG. 2, a size of a circle corresponding for each cell type may indicate population. For instance, a larger circle may indicate a larger population. The size may correspond to a percentage of cell population corresponding to the respective cell type, or may directly scale based on an amount of cells. In addition, the relative sizes between different circles may provide a visual estimate of relative sizes of the corresponding cell populations. Although not shown in FIG. 2, additional text, such as numerical values may be displayed as well.

Additional characteristics may be visually represented. Color or other markings may represent normal and/or abnormal ranges or results. For example, the characteristics may be represented by lines or dashed lines that may mark the transition from normal to abnormal, changes in the line weight connecting different cell types, changes of shape (e.g., using squares), changes in location in the chart, bars, other chart shapes, etc.

FIG. 2 shows the different cell types with a neutral color (e.g., gray) to indicate normal ranges. In other examples, other colors such as green may be used to display normal or lack of abnormal ranges.

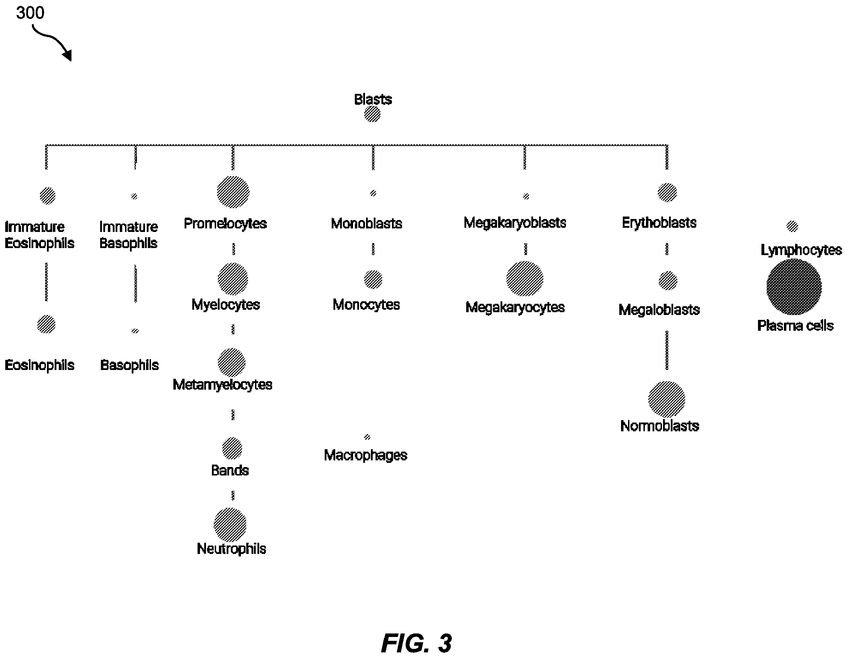

FIG. 3 shows a chart 300 of cell analysis similar to chart 200, except depicting a different result. Chart 300 may illustrate an abnormal bone marrow sample. For instance, chart 300 may illustrate an abnormality in plasma cells as indicated by a size of the corresponding circle as well as a color of the circle. In FIG. 3, the plasma cells may be represented by a dark color indicating abnormality or otherwise requiring attention. In other examples, abnormalities may be illustrated in warning colors such as red. Such visual indicators may advantageously provide a simple and intuitive representation of abnormalities.

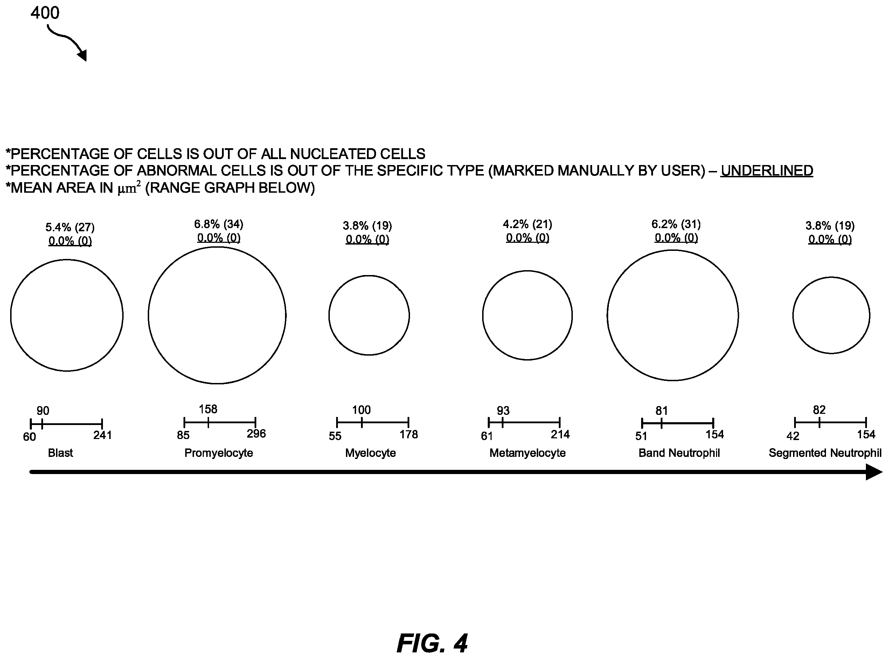

FIG. 4 illustrates an example chart 400 of another visual presentation of a cell lineage. Whereas charts 200 and 300 present various cell lineages (e.g., an extensive cell linage mapping), chart 400 may focus on a particular lineage (e.g., a column from charts 200 and 300). Similar to charts 200 and 300, chart 400 may display cell populations as circles with the sizes of the circles corresponding to cell populations, although in other examples different visual indicators may be used. Above the circles, text and/or numbers may present a percentage of cells of that type with respect to all nucleated cells and a percentage of abnormal cells (which may be manually marked by a user) of that type. In some examples, the text may be displayed in different colors, for instance neutral colors for the percentage out of all nucleated cells, and a red or warning color for the abnormal cells. Below the circles, ranges may present aggregated results such as mean areas (e.g. area covered by cells of that type, measured in µm²) between normal area values. As seen in chart 400, none of the mean area values lie outside of their respective normal ranges. Below the ranges, the cell types may be labeled, which may be presented in order of lineage progression as further indicated by the arrow.

Charts 200, 300, and 400 show specific example arrangements of visual indicators and data. In other examples, the charts may vary, for instance with different arrangements, different colors/symbols, different data, etc.

FIG. 5 illustrates an example screen 500 of a bone marrow aspirate sample analysis interface. As seen in FIG. 5, screen 500 shows image data 502 obtained from scanning an area of the sample. Visual indicators, such as color, may signify different information. For example, the darker portions may correspond to particles detected in the area. Certain information may be overlaid over the image data, including a boundary 504 and a boundary 506, which may be presented with different colors, line thicknesses, or other visual differences to distinguish from each other, for indicating elements detected in the sample. Boundary 504, illustrated with a dark line, may outline areas that have been computer analyzed (e.g., detecting particles, cells, etc.). Boundary 506, illustrated with a lighter line, may outline potential areas or regions of interest (ROI) that may be automatically determined based on computer analysis, user selected, etc. In other examples, other areas may be outlined with other boundaries.

Screen 500 may include a thumbnail 508 showing a preview image of the entire slide or a significant portion of the slide. Thumbnail 508 may have been imaged at a lower resolution and covering a wider area than that of image data 502. Thumbnail 508 may include a boundary 510 indicating which area of the sample that image data 502 corresponds to.

In some examples, screen 500 may include additional information displayed graphically or textually. For instance, statistical data (e.g., a total number of cells detected, a total number of intact cells detected, a total number of stripped cells detected, a total number of particles detected, a cellularity percentage, etc.) or other analysis results may be presented. In addition, user interface elements, such as controls for changing what data is displayed, adjusting or annotating data, selecting different areas or samples, continuing through a workflow, navigating to different areas, changing views, menus, etc. may be presented.

FIG. 6 illustrates an example screen 600 of a bone marrow aspirate sample analysis interface. As seen in FIG. 6, screen 600 shows image data 602 obtained from scanning an area of the sample. Image data 602 may comprise a cell heat map showing cell densities of different cell types. Visual indicators, such as color, may signify different information. For example, the different colors may correspond to different types of cells. The groupings of color points may indicate cell density. Certain information may be overlaid over the image data, including a highlight 604 which may be presented with different colors or other visual differences to distinguish from other highlights and visual indicators. Highlight 604, illustrated with a light gray shading over dense cell groupings, may indicate areas selected for further analysis such as areas that the system may suggest for nucleated differential cell count ("NDC") analysis. In other examples, different highlights may be used for indicating different cell groupings.

Screen 600 may include a thumbnail 608 showing a preview image of the entire slide or a significant portion of the slide. Thumbnail 608 may have been imaged at a lower resolution and covering a wider area than that of image data 602. Thumbnail 608 may include a boundary 610 indicating which area of the sample that image data 602 corresponds to.

In some examples, screen 600 may include additional information displayed graphically or textually. For instance, analysis results (e.g., numbers of cells of each detected type) or other statistical data may be presented. In addition, user interface elements, such as controls for changing what data is displayed, changing which highlights are displayed, adjusting or annotating data, selecting different samples, continuing through a workflow, navigating to different areas, changing views, menus, etc. may be presented.

FIG. 7 illustrates a flow chart of an exemplary method 700 for presenting bone marrow aspirate analysis. In one example, each of the steps shown in FIG. 7 may represent an algorithm whose structure includes and/or is represented by multiple sub-steps, examples of which will be provided in greater detail below. The steps shown in FIG. 7 may be performed by one or more of the systems and/or parts therein described herein, such as microscope 100 and/or parts therein (e.g., controller 106).

As illustrated in FIG. 7, at step 710 one or more of the systems described herein may obtain, using a scanning apparatus, scan data of a bone marrow aspirate ("BMA") sample. For example, microscope 100 may obtain, using image capture device 102, scan data of the BMA sample.

In some examples, image capture device 102 may have an effective numerical aperture ("NA") of at least 0.8. In some embodiments, the effective NA corresponds to a resolving power of the microscope that has the same resolving power as an objective lens with that NA. Image capture device 102 may also have an objective lens with a suitable magnification to provide the effective NA, although the NA of the objective lens may be less than the effective NA of the microscope.

In some examples, obtaining the scan data may include scanning an area of at least 0.5 square cm. For instance, the scan data may include at least 0.5 square cm of the BMA sample, at least 1 square cm of the BMA sample, at least 1.5 square cm of the BMA sample, or at least 2 square cm of the BMA sample. A scan time for obtaining the scan data may be faster than at least 20 minutes per square cm, 15 minutes per square cm, 10 minutes per square cm, 5 minutes per square cm, or 2 minutes per square cm. The scan data may include data from at least 95% of an entire area of the BMA sample.

In some examples, the scan data includes high-resolution image data of the BMA sample. The high-resolution image data may include image data of the BMA sample scanned with an effective NA of at least 0.8, image data of the BMA sample scanned with an effective NA of at least 0.9, and/or image data of the BMA sample scanned with an effective NA of at least 1.0.

In some examples, microscope 100 may analyze a preview image corresponding to the BMA sample to locate particles. The preview image may have a lower resolution than the scan data. Based on the analysis, microscope 100 may determine a recommended scan area for the BMA sample. While the preview image can be acquired in many ways, in some embodiments, the preview image is acquired with a camera without a microscope objective lens. For example, the preview image can be acquired with a macro lens or other suitable camera lens, without reliance on a microscope objective lens in order to acquire the preview image.

In some examples, the recommended scan area may be further determined based on a user input. The user input may include adjusting the recommended scan area to include or exclude a user-defined area.

In some examples, obtaining the scan data further may include scanning, using the scanning apparatus, the recommended scan area. For example, after the recommended scan area is finalized, microscope 100 may obtain the scan data by scanning, using image capture device 102, the recommended scan area.

At step 720 one or more of the systems described herein may detect cells in the BMA sample from the scan data. For example, microscope 100 may detect cells in the BMA sample from the scan data obtained in step 710. Microscope 100 may use, for instance, computer vision or other artificial intelligence ("AI"), such as machine learning. In some embodiments, the AI comprises image recognition processor instructions configured to detect cells and identify cell types.

In some examples, microscope 100 may detect particles in the BMA sample and detect cells in the BMA sample separate from the detected particles. The particles may comprise spicules or fragments, for example, and may include one or more of fat, or clusters of hematopoietic cells. In some examples, the detected cells comprise at least 100 cells. For instance, a number of the detected cells be at least 500 or more cells, 1000 or more cells, 2000 or more cells, 5000 or more cells, or 10000 or more cells.

At step 730 one or more of the systems described herein may classify the detected cells or a subset of the detected cells into a plurality of cell types. For example, microscope 100 may classify the detected cells into cell types.

In some examples, classifying the detected cells may further include assigning a cell lineage to cell types linking cells of different cell types and presenting the cell data further comprises presenting the cell lineage. The cell lineage may include a lineage of the cells from one or more of a myeloid lineage, a lymphoid lineage, or an erythroid lineage. FIGS. 2, 3, and 4 illustrate example lineages. In some examples, the number of classified cells may be at least 500 or more cells, 1000 or more cells, 2000 or more cells, 5000 or more cells, or 10000 or more cells.

In some examples, microscope 100 may classify the cells with a machine learning classifier. The machine learning classifier may include one or more models such as a neural network, a convolutional neural network, decision trees, support vector machines, regression analysis, Bayesian networks, and/or training models. The machine learning classifier may be configured to identify at least 10 cell types such as blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, and plasma cells.

At step 740 one or more of the systems described herein may store, in a memory, cell data of the classified cells. For example, microscope 100 may store the cell data in memory 108. Storing the cell data may allow modifying the cell data, and retrieving the cell data for presenting to the user.

In some examples, storing the cell data may further include selecting, from the detected cells, at least relevant cells to store in the cell data. The relevant cells may be selected based on a representative distribution of cell types from the detected cells. A number of the relevant cells may be between 100-500 cells, 500-1000 cells, 1000-2000 cells, or more than 2000 cells.

At step 750 one or more of the systems described herein may present the cell data. For example, microscope 100 may present the cell data using user interface 112.

Although the cell data may be presented as, for instance, table and/or textual readouts, the systems and methods described herein advantageously present the cell data graphically. For example, microscope 100 may display a cell density heat map (see, e.g., FIG. 6). FIGS. 2, 3, 4, and 5 show other examples of graphically presenting the cell data.

In some examples, microscope 100 may receive a user input to modify the cell data. The user input may include marking a cell, adding a cell, deleting a cell, reclassifying a cell, and/or adding a notification to a cell. The notification may indicate toxic or abnormal.

In some examples, illumination assembly 110 of microscope 100 may be configured to illuminate the BMA sample at various angles such that image capture device 102 may be configured to collect multiple images from the BMA sample illuminated by illumination assembly 110 at the various angles. The multiple images may comprise a first minimum resolvable distance for features of the cells.

Microscope 100 may (e.g., via controller 106 operatively coupled to illumination assembly 110 and image capture device 102) generate a computationally reconstructed image from the multiple images. The computationally reconstructed image may comprise a second minimum resolvable distance for the features of the cells. The second minimum resolvable distance may be less than the first minimum resolvable distance.

In some examples, microscope 100 may be configured to identify intact cells and determine an area for further analysis based on locations of the intact cells. For example, microscope 100 may identify intact cells from the preview image such that the area for further analysis may be the recommended scan area. Alternatively, microscope 100 may identify intact cells from the scan area such that the area for further analysis may be suggested for another scan, for analysis by the user, etc.

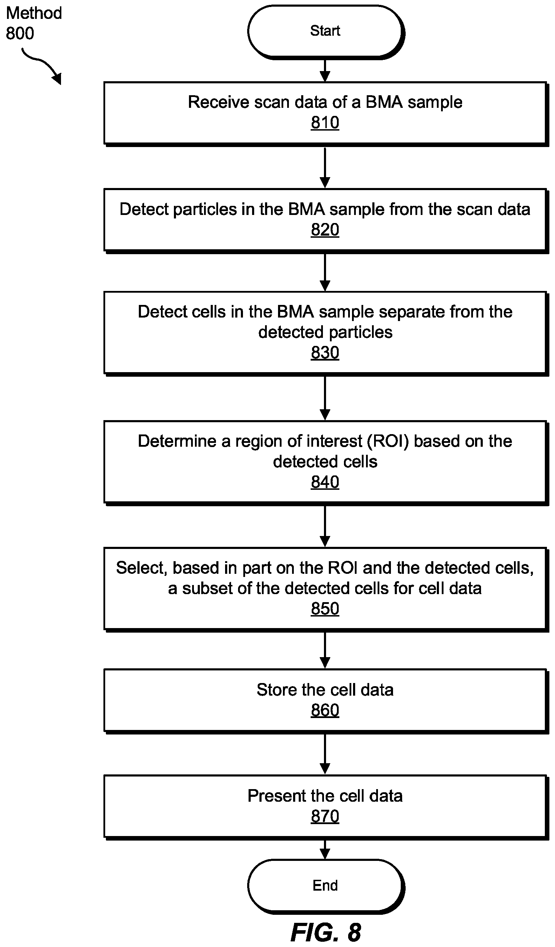

FIG. 8 illustrates a flow chart of an exemplary method 800 for presenting bone marrow aspirate analysis. In one example, each of the steps shown in FIG. 8 may represent an algorithm whose structure includes and/or is represented by multiple sub-steps, examples of which will be provided in greater detail below. The steps shown in FIG. 8 may be performed by one or more of the systems and/or parts therein described herein, such as microscope 100 and/or parts therein (e.g., controller 106).

As illustrated in FIG. 8, at step 810 one or more of the systems described herein may receive scan data of a BMA sample. For example, microscope 100 may receive the scan data of the BMA sample. Microscope 100 may have scanned the BMA sample itself, as described above, may receive the scan data from another scanning apparatus, or may receive data stored in a computing device.

At step 820 one or more of the systems described herein may detect particles in the BMA sample from the scan data. For example, microscope 100 may detect particles in the BMA sample. In some examples, microscope 100 may use computer vision or other AI-based image recognition to detect the particles, as described herein.

At step 830 one or more of the systems described herein may detect cells in the BMA sample separate from the detected particles. For example, microscope 100 may detect cells in the BMA sample separate from the detected particles. Microscope 100 may distinguish between particles and cells using, for example, computer vision or other AI-based image recognition.

In some examples, microscope 100 may detect intact cells. In some examples, microscope 100 may detect a cell density. In some examples, microscope 100 may classify cell types of the detected cells, as further described above.

At step 840 one or more of the systems described herein may determine a region of interest (ROI) based on the detected cells. For example, microscope 100 may determine the ROI based on the detected cells.

In some examples, the ROI may be further determined based on the detected intact cells. In some examples, the ROI may be further determined based on the cell density. For example, the ROI may be further determined based on distances between the detected cells and the detected particles. A desirable ROI may include intact cells, cells dispersed in a relevant density (e.g., not too dense or too sparse), and within a desired distance from particles.

In some examples, the ROI may be further determined based on relevant focal information corresponding to one or more specific locations of the BMA sample. For example, the relevant focal information may correspond to a focal disease with features at the one or more specific locations. The focal disease may comprise lymphoma, plasma cell myeloma, mastocytosis, metastatic carcinoma, storage histiocytosis, crystal storage histiocytosis, and/or granuloma, etc.

At step 850 one or more of the systems described herein may select, based in part on the ROI and the detected cells, a subset of the detected cells for cell data. For example, microscope 100 may select, based in part on the ROI and the detected cells, a subset of the detected cells for the cell data.

In some examples, the ROI may comprise multiple ROIs. Each ROI may include a distribution of cells. The subset of cells may be selected from a subset of the multiple ROIs based on a comparison of a collective distribution of cells from the multiple ROIs to a total distribution of cells from the ROI.

In some examples, the subset of cells may be further selected based on having a cell distribution of cell types representative of a total cell distribution of cell types in the scan data. The subset of cells may be further selected based on prioritizing one or more cell types for inclusion into the cell data. For example, the prioritized cell types may include blast cells and/or plasma cells.

In some embodiments, either or both of the following approaches is used to select the subset of selected cells as cell data for presentation:
1. A subset of the ROIs is chosen, and the cells present in the subset of ROIs is selected. These ROIs can be chosen in response to the aggregated set of cells in these ROIs representing the distribution of cells in the sample. In some embodiments, this also includes evaluation to ensure that specific cell types are well represented in the selected subset of ROIs.
2. In some embodiments, a subset of cells from the general cell population of the sample is chosen to represent the statistics of the cells from the general population. For example, the subset of cells can be chosen to maintain percentages corresponding to the general population, and these chosen cells may not include all cells from an ROI that contributed cells to the subset. Alternatively or in combination, the subset of cells may be chosen to prioritize one or more focal diseases, or to prioritize certain cell types as described herein. In some embodiments, the one or more focal diseases corresponds to many cells of specific types or distributions in a specific area of the sample.

Work in relation to the present disclosure suggests that it can be helpful for a user to confirm that the subset of cells was generated without substantial bias. In some embodiments, the processor is configured with instructions to generate a second subset of cells to provide data to the user that may be helpful in detecting bias. In some embodiments, a second subset of the identified cells comprises randomly selected cells of one or more cell types that were not chosen for the subset of identified cells. The data from the second subset can be presented to a user to allow the user to detect bias in the subset.

In some embodiments, the potential for bias (if present) can be reduced by showing a random assortment of cells selected for the second subset that were not chosen as part of the subset of cells. For example, if the AI process as described herein were to systematically not choose something that the user is interested in, e.g. certain cell types, the random selection process will enable the user to notice this bias in the selection process when data from the second subset of cells is presented.

At step 860 one or more of the systems described herein may store, in the memory, the cell data. For example, microscope 100 may store the cell data in memory 108. The cell data may be stored for modifying and/or retrieving for presentation.

At step 870 one or more of the systems described herein may present the cell data. For example, microscope 100 may present the cell data using user interface 112.

In some examples, presenting the cell data may include presenting a comparison of cell types of a first set of cells to a second set of cells. In some examples, the comparison may include a ratio of the first set of cells to the second set of cells. The first set of cells may correspond to the selected subset of cells. The second set of cells may correspond to a general population of cells from the BMA sample. For example, FIG. 4 illustrates the comparison as a percentage of cell type out of all nucleated cells.

In some examples, microscope 100 may display a cell density heat map (see, e.g., FIG. 6). In some examples, microscope 100 may display other graphical presentations (see, e.g., FIGS. 2, 3, and 5).

In some examples, microscope 100 may edit the cell data based on a user input, for example user inputs as described above.

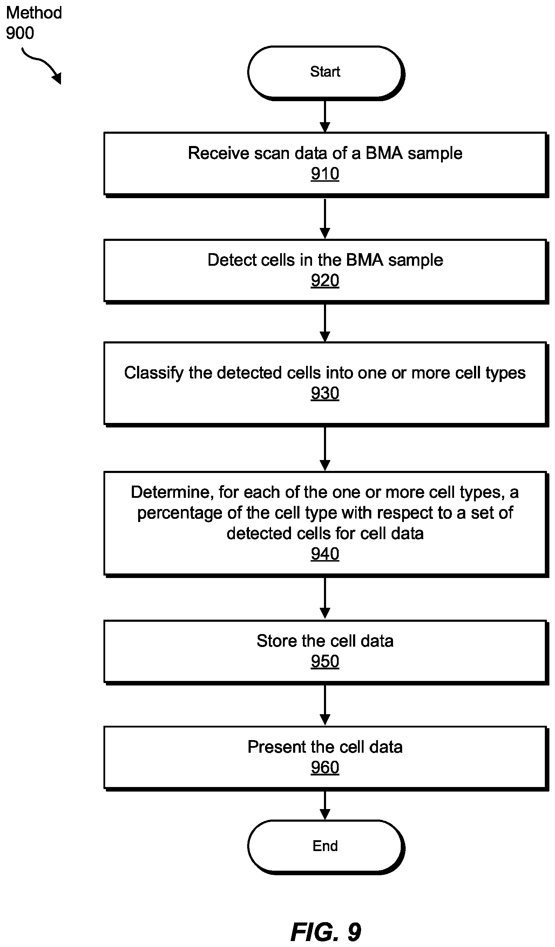

FIG. 9 illustrates a flow chart of an exemplary method 900 for presenting bone marrow aspirate analysis. In one example, each of the steps shown in FIG. 9 may represent an algorithm whose structure includes and/or is represented by multiple sub-steps, examples of which will be provided in greater detail below. The steps shown in FIG. 9 may be performed by one or more of the systems and/or parts therein described herein, such as microscope 100 and/or parts therein (e.g., controller 106).

As illustrated in FIG. 9, at step 910 one or more of the systems described herein may receive scan data of the BMA sample. For example, microscope 100 may receive scan data of the BMA sample. In some examples, microscope 100 may receive the scan data as described above.

At step 920 one or more of the systems described herein may detect cells in the BMA sample. For example, microscope 100 may detect cells in the BMA sample. In some examples, microscope 100 may detect the cells using computer vision or other AI-based image recognition, as described above.

At step 930 one or more of the systems described herein may classify the detected cells into one or more cell types. For example, microscope 100 may classify the detected cells into cell types. Microscope 100 may classify the cells using a machine learning classifier running on a processor, as described herein. In some examples, microscope 100 may classify the cells into cell lineages, as described herein.

At step 940 one or more of the systems described herein may determine, for each of the one or more cell types, a percentage of the cell type with respect to a set of detected cells for cell data. For example, the processor of microscope 100 may determine the percentage of each cell type with respect to the set of detected cells.

In some examples, the set of detected cells corresponds to a general population of the detected cells or a chosen subset of the detected cells. FIG. 4 illustrates the percentage with respect to the general population. Alternatively or in combination, the set of detected cells may comprise a lineage of cells, in which the cell data comprises the proportions of cell types within the lineage with respect to other cells of the lineage, such as a percentage of each cell type within the lineage. In some embodiments, the cell data may comprise relative proportions of cells that are helpful for a system user to make a diagnosis, such as the best cells to present for the user to make a diagnosis.

In some examples, microscope 100 may perform further analysis to store additional cell data, as described herein. For example, the cell data may include a percentage of abnormal cells for each of the one or more cell types (see, e.g., FIG. 4). The cell data may include statistical analysis for each of the one or more cell types. For instance, the statistical analysis includes at least one of a distribution, an average, or a median (see, e.g., FIG. 4). In some embodiments, the statistical analysis includes one or more of a distribution, an average, or a median with respect to reference values, such as a range of reference values for each cell type.

At step 950 one or more of the systems described herein may store, in the memory, the cell data. For example, microscope 100 may store the cell data in memory 108.

At step 960 one or more of the systems described herein may present the cell data based on a lineage of the one or more cell types. For example, microscope 100 may present, using user interface 112, the cell data based on cell lineage.

In some examples, presenting the cell data may include a graphic presentation of the lineage of the one or more cell types (see, e.g., FIG. 4). The lineage may comprise a lineage of the cells from a myeloid lineage, a lymphoid lineage, and/or an erythroid lineage.

In some examples, the graphic presentation may include, for each of the one or more cell types, a visual comparison of the percentage of the cell type with respect to the set of detected cells or with respect to a subset of the set of detected cells (see, e.g., FIG. 4). In some examples, microscope 100 may receive a user input to select the subset of detected cells. For example, microscope 100 may receive a user input to compare the cell type with cells in a particular area, general population, etc.

In some examples, the visual comparison may include, for each of the one or more cell types, a shape that is scaled based on the percentage of the cell type (see, e.g., FIGS. 2, 3, and 4). In some examples, the graphic presentation includes, for each of the one or more cell types, a visual comparison based on a range of values (see, e.g., FIG. 4). In some examples, the graphic presentation may include one or more colors corresponding to one or more cell properties. In some examples, presenting the cell data includes presenting a cell density heat map (see, e.g., FIG. 6).

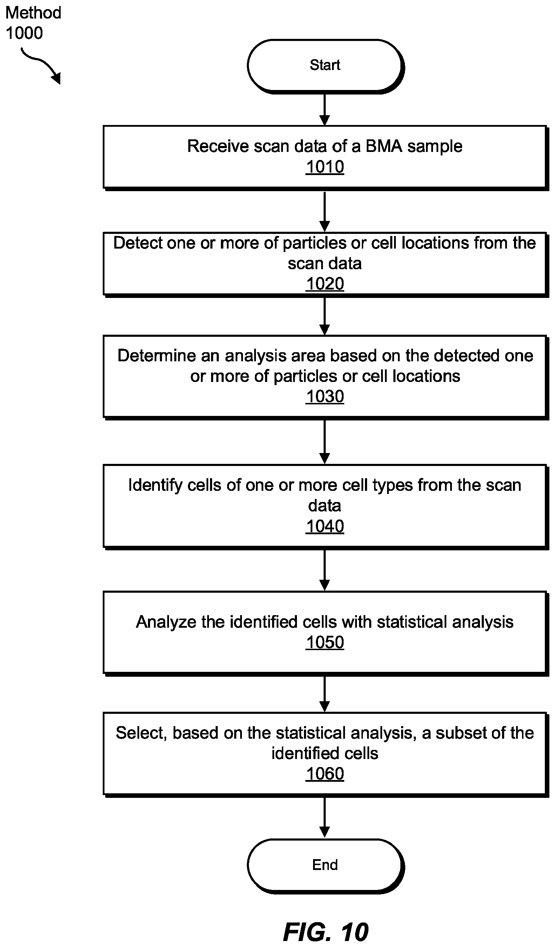

FIG. 10 illustrates a flow chart of an exemplary method 1000 for presenting bone marrow aspirate analysis. In one example, each of the steps shown in FIG. 10 may represent an algorithm whose structure includes and/or is represented by multiple sub-steps, examples of which will be provided in greater detail below. The steps shown in FIG. 10 may be performed by one or more of the systems and/or parts therein described herein, such as microscope 100 and/or parts therein (e.g., controller 106).

As illustrated in FIG. 10, at step 1010 one or more of the systems described herein may receive scan data from the BMA sample. For example, microscope 100 may receive scan data from the BMA sample. Microscope 100 may receive the scan data from image capture device 102 or another device, as described above.

At step 1020 one or more of the systems described herein may detect one or more of particles or cell locations from the scan data. For example, microscope 100 may detect particles and/or cell locations from the scan data. Microscope 100 may detect the particles and/or cell locations using computer vision or other AI-based image recognition, as described above.

At step 1030 one or more of the systems described herein may determine an analysis area based on the detected one or more of particles or cell locations. For example, microscope 100 may determine the analysis area based on the detected particles and/or cell locations. In some examples, determining the analysis area may further comprise determining relevant locations based on identifying intact cells. For example, microscope 100 may detect, using computer vision or other AI-based image recognition, intact cells and accordingly determine the analysis area. In some examples, determining the analysis area may be similar to determining an ROI as described above.

At step 1040 one or more of the systems described herein may identify cells of one or more cell types from the scan data. For example, microscope 100 may identify cells of one or more cell types from the scan data.

At step 1050 one or more of the systems described herein may analyze the identified cells with statistical analysis. For example, microscope 100 may analyze the identified cells with statistical analysis.

In some examples, the statistical analysis may be based on locations of the identified cells. In some examples, the statistical analysis may be based on comparing cell properties with respect to a general population of the identified cells.

In some examples, the statistical analysis may be adjusted based on a user input. For example, the user input may include adjusting the recommended scan area to include or exclude a user-defined area.

At step 1060 one or more of the systems described herein may select, based on the statistical analysis, a subset of the identified cells. For example, microscope 100 may select the subset of the identified cells based on the statistical analysis. By selecting the subset of cells from the analysis area, a good selection of representative cells may be selected without having to analyze most of the general population of cells. For example, instead of having to analyze 1000s of cells, 100s of cells may be analyzed while achieving a requisite sensitivity for analyzing the sample.

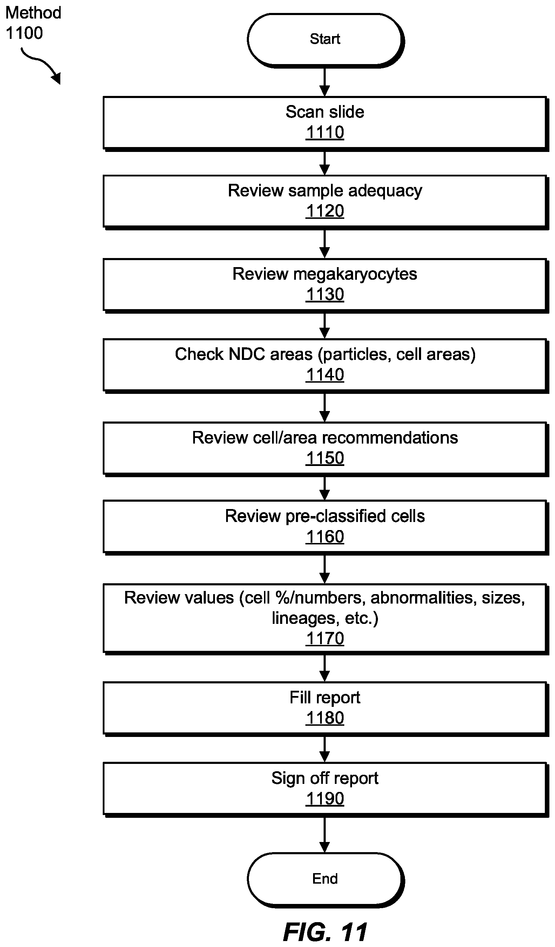

FIG. 11 illustrates a flow chart of an exemplary method 1100 for a user reviewing bone marrow aspirate analysis. In one example, each of the steps shown in FIG. 11 may represent an algorithm whose structure includes and/or is represented by multiple sub-steps, examples of which will be provided in greater detail below.

As illustrated in FIG. 11, at step 1110 one or more of the systems described herein may scan a slide. At step 1120 one or more of the systems described herein may review a sample adequacy. At step 1130 one or more of the systems described herein may review megakaryocytes. At step 1140 one or more of the systems described herein may check NDC areas, such as particles, cell areas, etc. At step 1150 one or more of the systems described herein may review cell and/or area recommendations. At step 1160 one or more of the systems described herein may review pre-classified cells. At step 1170 one or more of the systems described herein may review values, such as cell percentages and/or numbers, abnormalities, sizes, lineages, etc. The values may be derived from, for instance, statistical analysis as described herein. At step 1180 one or more of the systems described herein may fill a report. For example, a user may review cell data (e.g., as a result of computer analysis as described herein), make adjustments, notes, etc., and fill the report. At step 1190 one or more of the systems described herein may sign off the report. For example, the user may sign off the report to finalize it. The user may have the requisite permission (e.g., certification, experience, etc.) for signing off on the report. In some examples, the user signing off the report may be different from the user filling the report such that the user signing off the report may verify the accuracy of the report before finalizing it.

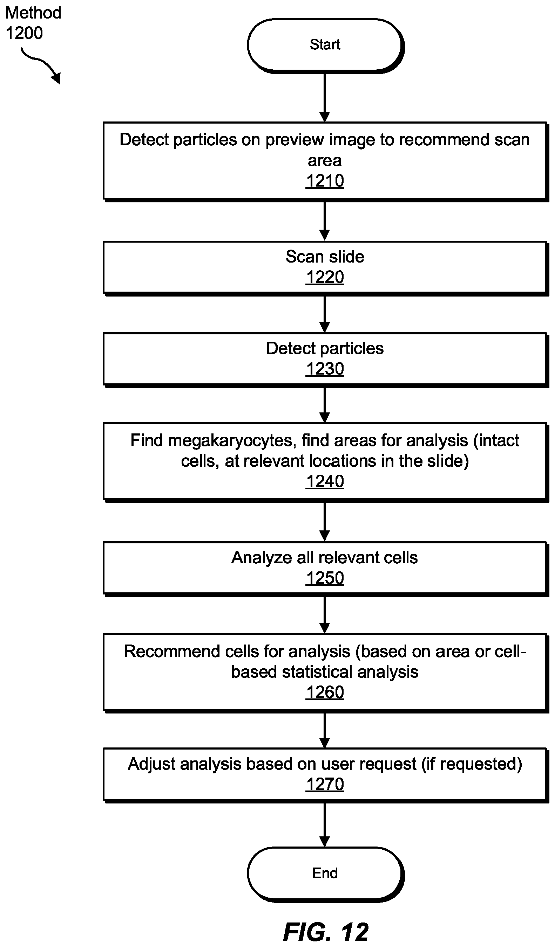

FIG. 12 illustrates a flow chart of an exemplary method 1200 for AI-based bone marrow aspirate analysis. In one example, each of the steps shown in FIG. 12 may represent an algorithm whose structure includes and/or is represented by multiple sub-steps, examples of which will be provided in greater detail below. The steps shown in FIG. 12 may be performed by one or more of the systems and/or parts therein described herein, such as microscope 100 and/or parts therein (e.g., controller 106).

As illustrated in FIG. 12, at step 1210 one or more of the systems described herein may detect particles on preview image of a slide to recommend a scan area of the slide. At step 1220 one or more of the systems described herein may scan the slide. At step 1230 one or more of the systems described herein may detect particles from the scan. At step 1240 one or more of the systems described herein may find megakaryocytes and find areas for analysis. For example, the areas may be based on intact cells at relevant locations in the slide. At step 1250 one or more of the systems described herein may analyze all relevant cells, such as cells within the areas for analysis. At step 1260 one or more of the systems described herein may recommend cells for analysis. The recommendation may be, for instance, based on area and/or cell-based statistical analysis. At step 1270 one or more of the systems described herein may adjust the analysis based on user request, if requested by a user. For example, the user may select different areas and/or cells, request different types of analysis, request rescanning or scanning of different areas, etc.

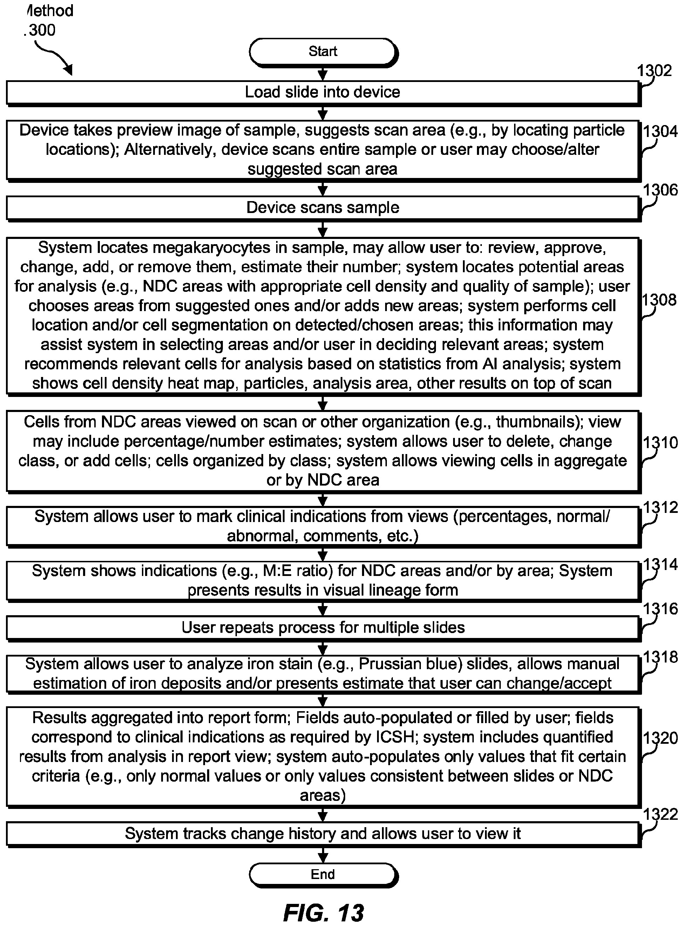

FIG. 13 illustrates a flow chart of an exemplary method 1300 for presenting bone marrow aspirate analysis. In one example, each of the steps shown in FIG. 13 may represent an algorithm whose structure includes and/or is represented by multiple sub-steps, examples of which will be provided in greater detail below. The steps shown in FIG. 13 may be performed by one or more of the systems and/or parts therein described herein, such as microscope 100 and/or parts therein (e.g., controller 106).

As illustrated in FIG. 13, at step 1302 one or more of the systems described herein may load a slide into a device. For example, a bone marrow aspirate sample may be mounted on a slide that is loaded into microscope 100. At step 1304 one or more of the systems described herein may take a preview image of the sample and suggest a scan area (e.g., by locating particle locations). As illustrated in FIG. 13, at step 1306 one or more of the systems described herein may scan the sample, for instance scanning the suggested scan area.

At step 1308 one or more of the systems described herein may locate megakaryocytes in sample. For example, microscope 100 may allow user to: review, approve, change, add, or remove detected megakaryocytes from analysis, estimate their number, etc. Microscope 100 may locate potential areas for analysis (e.g., NDC areas with appropriate cell density and quality of sample). The user may choose areas from suggested ones and/or adds new areas. Microscope 100 may perform cell location and/or cell segmentation on the detected/chosen areas. This information may assist microscope 100 in selecting areas and/or assist the user in deciding relevant areas. Microscope 100 may recommend relevant cells for analysis based on statistics from AI analysis. Microscope 100 may show a cell density heat map (see, e.g., FIG. 6), particles, analysis area, other results on top of scan (see, e.g., FIG. 5).

At step 1310 one or more of the systems described herein may view cells from NDC areas via scan image or other organization (e.g., thumbnails). The view may include percentage/number estimates. Microscope 100 may allow the user to delete, change class, or add cells. The cells may be organized by class (see, e.g., FIGS. 2, 3, and 4). Microscope 100 may allow viewing cells in aggregate or by NDC area.

At step 1312 one or more of the systems described herein may allow the user to mark clinical indications from views (percentages, normal/abnormal, comments, etc.). At step 1314 one or more of the systems described herein may show indications (e.g., M:E ratio) for NDC areas and/or by area. For example, microscope 100 may presents results in visual lineage form (see, e.g., FIGS. 2, 3, and 4).

At step 1316 one or more of the systems described herein may allow the user to repeat this process for multiple slides. At step 1318 one or more of the systems described herein may allow the user to analyze iron stain (e.g., Prussian blue) slides. For instance, microscope 100 may allow manual estimation of iron deposits and/or present estimate that user may change and/or accept.

At step 1320 one or more of the systems described herein may aggregate the results into report form. For example, microscope 100 may auto-populate fields or allow the user to fill the fields. The fields may correspond to clinical indications as required by ICSH. Microscope 100 may include quantified results from the analysis in report view and may auto-populate only values that fit certain criteria (e.g., only normal values or only values consistent between slides or NDC areas).

At step 1322 one or more of the systems described herein may track a change history and allows user to view it.

Although various methods are described above with respect to separate flowcharts, in some examples, one or more steps from the various methods may be combined, performed in different order, and/or repeated. In addition, in certain examples, different users (e.g., users with different permissions, different expertise, etc.) may perform different steps of the methods described herein.

FIG. 14 is a block diagram of an example computing system 1410 capable of implementing one or more of the embodiments described and/or illustrated herein. For example, all or a portion of computing system 1410 may perform and/or be a means for performing, either alone or in combination with other elements, one or more of the steps described herein (such as one or more of the steps illustrated in FIGS. 7, 8, 9, 10, 11, 12, and 13). All or a portion of computing system 1410 may also perform and/or be a means for performing any other steps, methods, or processes described and/or illustrated herein. All or a portion of computing system 1410 may correspond to or otherwise be integrated with microscope 100 (e.g., one or more of controller 106, memory 108, and/or user interface 112).

Computing system 1410 broadly represents any single or multi-processor computing device or system capable of executing computer-readable instructions. Examples of computing system 1410 include, without limitation, workstations, laptops, client-side terminals, servers, distributed computing systems, handheld devices, or any other computing system or device. In its most basic configuration, computing system 1410 may include at least one processor 1414 and a system memory 1416.

Processor 1414 generally represents any type or form of physical processing unit (e.g., a hardware-implemented central processing unit) capable of processing data or interpreting and executing instructions. In certain embodiments, processor 1414 may receive instructions from a software application or module. These instructions may cause processor 1414 to perform the functions of one or more of the example embodiments described and/or illustrated herein.

System memory 1416 generally represents any type or form of volatile or non-volatile storage device or medium capable of storing data and/or other computer-readable instructions. Examples of system memory 1416 include, without limitation, Random Access Memory (RAM), Read Only Memory (ROM), flash memory, or any other suitable memory device. Although not required, in certain embodiments computing system 1410 may include both a volatile memory unit (such as, for example, system memory 1416) and a non-volatile storage device (such as, for example, primary storage device 1432, as described in detail below). In one example, one or more of steps from FIGS. 7, 8, 9, 10, 11, 12, and 13 may be computer instructions that may be loaded into system memory 1416.

In some examples, system memory 1416 may store and/or load an operating system 1440 for execution by processor 1414. In one example, operating system 1440 may include and/or represent software that manages computer hardware and software resources and/or provides common services to computer programs and/or applications on computing system 1410. Examples of operating system 1440 include, without limitation, LINUX, JUNOS, MICROSOFT WINDOWS, WINDOWS MOBILE, MAC OS, APPLE'S IOS, UNIX, GOOGLE CHROME OS, GOOGLE'S ANDROID, SOLARIS, variations of one or more of the same, and/or any other suitable operating system.

In certain embodiments, example computing system 1410 may also include one or more components or elements in addition to processor 1414 and system memory 1416. For example, as illustrated in FIG. 14, computing system 1410 may include a memory controller 1418, an Input/Output (I/O) controller 1420, and a communication interface 1422, each of which may be interconnected via a communication infrastructure 1412. Communication infrastructure 1412 generally represents any type or form of infrastructure capable of facilitating communication between one or more components of a computing device. Examples of communication infrastructure 1412 include, without limitation, a communication bus (such as an Industry Standard Architecture (ISA), Peripheral Component Interconnect (PCI), PCI Express (PCIe), or similar bus) and a network.

Memory controller 1418 generally represents any type or form of device capable of handling memory or data or controlling communication between one or more components of computing system 1410. For example, in certain embodiments memory controller 1418 may control communication between processor 1414, system memory 1416, and I/O controller 1420 via communication infrastructure 1412.

I/O controller 1420 generally represents any type or form of module capable of coordinating and/or controlling the input and output functions of a computing device. For example, in certain embodiments I/O controller 1420 may control or facilitate transfer of data between one or more elements of computing system 1410, such as processor 1414, system memory 1416, communication interface 1422, display adapter 1426, input interface 1430, and storage interface 1434.

As illustrated in FIG. 14, computing system 1410 may also include at least one display device 1424 (which may correspond to user interface 112) coupled to I/O controller 1420 via a display adapter 1426. Display device 1424 generally represents any type or form of device capable of visually displaying information forwarded by display adapter 1426. Similarly, display adapter 1426 generally represents any type or form of device configured to forward graphics, text, and other data from communication infrastructure 1412 (or from a frame buffer, as known in the art) for display on display device 1424.

As illustrated in FIG. 14, example computing system 1410 may also include at least one input device 1428 (which may correspond to user interface 112) coupled to I/O controller 1420 via an input interface 1430. Input device 1428 generally represents any type or form of input device capable of providing input, either computer or human generated, to example computing system 1410. Examples of input device 1428 include, without limitation, a keyboard, a pointing device, a speech recognition device, variations or combinations of one or more of the same, and/or any other input device.

Additionally or alternatively, example computing system 1410 may include additional I/O devices. For example, example computing system 1410 may include I/O device 1436. In this example, I/O device 1436 may include and/or represent a user interface that facilitates human interaction with computing system 1410. Examples of I/O device 1436 include, without limitation, a computer mouse, a keyboard, a monitor, a printer, a modem, a camera, a scanner, a microphone, a touchscreen device, variations or combinations of one or more of the same, and/or any other I/O device.

Communication interface 1422 broadly represents any type or form of communication device or adapter capable of facilitating communication between example computing system 1410 and one or more additional devices. For example, in certain embodiments communication interface 1422 may facilitate communication between computing system 1410 and a private or public network including additional computing systems. Examples of communication interface 1422 include, without limitation, a wired network interface (such as a network interface card), a wireless network interface (such as a wireless network interface card), a modem, and any other suitable interface. In at least one embodiment, communication interface 1422 may provide a direct connection to a remote server via a direct link to a network, such as the Internet. Communication interface 1422 may also indirectly provide such a connection through, for example, a local area network (such as an Ethernet network), a personal area network, a telephone or cable network, a cellular telephone connection, a satellite data connection, or any other suitable connection.

In certain embodiments, communication interface 1422 may also represent a host adapter configured to facilitate communication between computing system 1410 and one or more additional network or storage devices via an external bus or communications channel. Examples of host adapters include, without limitation, Small Computer System Interface (SCSI) host adapters, Universal Serial Bus (USB) host adapters, Institute of Electrical and Electronics Engineers (IEEE) 1394 host adapters, Advanced Technology Attachment (ATA), Parallel ATA (PATA), Serial ATA (SATA), and External SATA (eSATA) host adapters, Fibre Channel interface adapters, Ethernet adapters, or the like. Communication interface 1422 may also allow computing system 1410 to engage in distributed or remote computing. For example, communication interface 1422 may receive instructions from a remote device or send instructions to a remote device for execution.

In some examples, system memory 1416 may store and/or load a network communication program 1438 for execution by processor 1414. In one example, network communication program 1438 may include and/or represent software that enables computing system 1410 to establish a network connection 1442 with another computing system (not illustrated in FIG. 14) and/or communicate with the other computing system by way of communication interface 1422. In this example, network communication program 1438 may direct the flow of outgoing traffic that is sent to the other computing system via network connection 1442. Additionally or alternatively, network communication program 1438 may direct the processing of incoming traffic that is received from the other computing system via network connection 1442 in connection with processor 1414.

Although not illustrated in this way in FIG. 14, network communication program 1438 may alternatively be stored and/or loaded in communication interface 1422. For example, network communication program 1438 may include and/or represent at least a portion of software and/or firmware that is executed by a processor and/or Application Specific Integrated Circuit (ASIC) incorporated in communication interface 1422.

As illustrated in FIG. 14, example computing system 1410 may also include a primary storage device 1432 and a backup storage device 1433 coupled to communication infrastructure 1412 via a storage interface 1434. Storage devices 1432 and 1433 generally represent any type or form of storage device or medium capable of storing data and/or other computer-readable instructions. For example, storage devices 1432 and 1433 may be a magnetic disk drive (e.g., a so-called hard drive), a solid state drive, a floppy disk drive, a magnetic tape drive, an optical disk drive, a flash drive, or the like. Storage interface 1434 generally represents any type or form of interface or device for transferring data between storage devices 1432 and 1433 and other components of computing system 1410. In one example, scan data 1435 (which may correspond to the scan data described herein) and/or cell data 1437 (which may correspond to the cell data described herein) may be stored and/or loaded in primary storage device 1432.

In certain embodiments, storage devices 1432 and 1433 may be configured to read from and/or write to a removable storage unit configured to store computer software, data, or other computer-readable information. Examples of suitable removable storage units include, without limitation, a floppy disk, a magnetic tape, an optical disk, a flash memory device, or the like. Storage devices 1432 and 1433 may also include other similar structures or devices for allowing computer software, data, or other computer-readable instructions to be loaded into computing system 1410. For example, storage devices 1432 and 1433 may be configured to read and write software, data, or other computer-readable information. Storage devices 1432 and 1433 may also be a part of computing system 1410 or may be a separate device accessed through other interface systems.

Many other devices or subsystems may be connected to computing system 1410. Conversely, all of the components and devices illustrated in FIG. 14 need not be present to practice the embodiments described and/or illustrated herein. The devices and subsystems referenced above may also be interconnected in different ways from that shown in FIG. 14. Computing system 1410 may also employ any number of software, firmware, and/or hardware configurations. For example, one or more of the example embodiments disclosed herein may be encoded as a computer program (also referred to as computer software, software applications, computer-readable instructions, or computer control logic) on a computer-readable medium. The term "computer-readable medium," as used herein, generally refers to any form of device, carrier, or medium capable of storing or carrying computer-readable instructions. Examples of computer-readable media include, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

The computer-readable medium containing the computer program may be loaded into computing system 1410. All or a portion of the computer program stored on the computer-readable medium may then be stored in system memory 1416 and/or various portions of storage devices 1432 and 1433. When executed by processor 1414, a computer program loaded into computing system 1410 may cause processor 1414 to perform and/or be a means for performing the functions of one or more of the example embodiments described and/or illustrated herein. Additionally or alternatively, one or more of the example embodiments described and/or illustrated herein may be implemented in firmware and/or hardware. For example, computing system 1410 may be configured as an Application Specific Integrated Circuit (ASIC) adapted to implement one or more of the example embodiments disclosed herein.

FIG. 15 is a block diagram of an example network architecture 1500 in which client systems 1510, 1520, and 1530 and servers 1540 and 1545 may be coupled to a network 1550. As detailed above, all or a portion of network architecture 1500 may perform and/or be a means for performing, either alone or in combination with other elements, one or more of the steps disclosed herein (such as one or more of the steps illustrated in FIGS. 7, 8, 9, 10, 11, 12, and 13). All or a portion of network architecture 1500 may also be used to perform and/or be a means for performing other steps and features set forth in the instant disclosure.

Client systems 1510, 1520, and 1530 generally represent any type or form of computing device or system, such as example computing system 1410 in FIG. 14. Similarly, servers 1540 and 1545 generally represent computing devices or systems, such as application servers or database servers, configured to provide various database services and/or run certain software applications. Network 1550 generally represents any telecommunication or computer network including, for example, an intranet, a WAN, a LAN, a PAN, or the Internet. In one example, client systems 1510, 1520, and/or 1530 and/or servers 1540 and/or 1545 may include all or a portion of microscope 100 from FIG. 1.

As illustrated in FIG. 15, one or more storage devices 1560(1)-(N) may be directly attached to server 1540. Similarly, one or more storage devices 1570(1)-(N) may be directly attached to server 1545. Storage devices 1560(1)-(N) and storage devices 1570(1)-(N) generally represent any type or form of storage device or medium capable of storing data and/or other computer-readable instructions. In certain embodiments, storage devices 1560(1)-(N) and storage devices 1570(1)-(N) may represent Network-Attached Storage (NAS) devices configured to communicate with servers 1540 and 1545 using various protocols, such as Network File System (NFS), Server Message Block (SMB), or Common Internet File System (CIFS).

Servers 1540 and 1545 may also be connected to a Storage Area Network (SAN) fabric 1580. SAN fabric 1580 generally represents any type or form of computer network or architecture capable of facilitating communication between a plurality of storage devices. SAN fabric 1580 may facilitate communication between servers 1540 and 1545 and a plurality of storage devices 1590(1)-(N) and/or an intelligent storage array 1595. SAN fabric 1580 may also facilitate, via network 1550 and servers 1540 and 1545, communication between client systems 1510, 1520, and 1530 and storage devices 1590(1)-(N) and/or intelligent storage array 1595 in such a manner that devices 1590(1)-(N) and array 1595 appear as locally attached devices to client systems 1510, 1520, and 1530. As with storage devices 1560(1)-(N) and storage devices 1570(1)-(N), storage devices 1590(1)-(N) and intelligent storage array 1595 generally represent any type or form of storage device or medium capable of storing data and/or other computer-readable instructions.

In certain embodiments, and with reference to example computing system 1410 of FIG. 14, a communication interface, such as communication interface 1422 in FIG. 14, may be used to provide connectivity between each client system 1510, 1520, and 1530 and network 1550. Client systems 1510, 1520, and 1530 may be able to access information on server 1540 or 1545 using, for example, a web browser or other client software. Such software may allow client systems 1510, 1520, and 1530 to access data hosted by server 1540, server 1545, storage devices 1560(1)-(N), storage devices 1570(1)-(N), storage devices 1590(1)-(N), or intelligent storage array 1595. Although FIG. 15 depicts the use of a network (such as the Internet) for exchanging data, the embodiments described and/or illustrated herein are not limited to the Internet or any particular network-based environment.

In at least one embodiment, all or a portion of one or more of the example embodiments disclosed herein may be encoded as a computer program and loaded onto and executed by server 1540, server 1545, storage devices 1560(1)-(N), storage devices 1570(1)-(N), storage devices 1590(1)-(N), intelligent storage array 1595, or any combination thereof. All or a portion of one or more of the example embodiments disclosed herein may also be encoded as a computer program, stored in server 1540, run by server 1545, and distributed to client systems 1510, 1520, and 1530 over network 1550.

As detailed above, computing system 1410 and/or one or more components of network architecture 1500 may perform and/or be a means for performing, either alone or in combination with other elements, one or more steps of an example method for bone marrow aspirate analysis.

As described herein, the computing devices and systems described and/or illustrated herein broadly represent any type or form of computing device or system capable of executing computer-readable instructions, such as those contained within the modules described herein. In their most basic configuration, these computing device(s) may each comprise at least one memory device and at least one physical processor.

The term "memory" or "memory device," as used herein, generally represents any type or form of volatile or non-volatile storage device or medium capable of storing data and/or computer-readable instructions. In one example, a memory device may store, load, and/or maintain one or more of the modules described herein. Examples of memory devices comprise, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In addition, the term "processor" or "physical processor," as used herein, generally refers to any type or form of hardware-implemented processing unit capable of interpreting and/or executing computer-readable instructions. In one example, a physical processor may access and/or modify one or more modules stored in the above-described memory device. Examples of physical processors comprise, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor. The processor may comprise a distributed processor system, e.g. running parallel processors, or a remote processor such as a server, and combinations thereof.

Although illustrated as separate elements, the method steps described and/or illustrated herein may represent portions of a single application. In addition, in some embodiments one or more of these steps may represent or correspond to one or more software applications or programs that, when executed by a computing device, may cause the computing device to perform one or more tasks, such as the method step.

In addition, one or more of the devices described herein may transform data, physical devices, and/or representations of physical devices from one form to another. Additionally or alternatively, one or more of the modules recited herein may transform a processor, volatile memory, non-volatile memory, and/or any other portion of a physical computing device from one form of computing device to another form of computing device by executing on the computing device, storing data on the computing device, and/or otherwise interacting with the computing device.

The term "computer-readable medium," as used herein, generally refers to any form of device, carrier, or medium capable of storing or carrying computer-readable instructions. Examples of computer-readable media comprise, without limitation, transmission-type media, such as carrier waves, and non-transitory-type media, such as magnetic-storage media (e.g., hard disk drives, tape drives, and floppy disks), optical-storage media (e.g., Compact Disks (CDs), Digital Video Disks (DVDs), and BLU-RAY disks), electronic-storage media (e.g., solid-state drives and flash media), and other distribution systems.

A person of ordinary skill in the art will recognize that any process or method disclosed herein can be modified in many ways. The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed.

The various exemplary methods described and/or illustrated herein may also omit one or more of the steps described or illustrated herein or comprise additional steps in addition to those disclosed. Further, a step of any method as disclosed herein can be combined with any one or more steps of any other method as disclosed herein.

The processor as described herein can be configured to perform one or more steps of any method disclosed herein. Alternatively or in combination, the processor can be configured to combine one or more steps of one or more methods as disclosed herein.

Unless otherwise noted, the terms "connected to" and "coupled to" (and their derivatives), as used in the specification and claims, are to be construed as permitting both direct and indirect (i.e., via other elements or components) connection. In addition, the terms "a" or "an," as used in the specification and claims, are to be construed as meaning "at least one of." Finally, for ease of use, the terms "including" and "having" (and their derivatives), as used in the specification and claims, are interchangeable with and shall have the same meaning as the word "comprising.

The processor as disclosed herein can be configured with instructions to perform any one or more steps of any method as disclosed herein.

It will be understood that although the terms "first," "second," "third", etc. may be used herein to describe various layers, elements, components, regions or sections without referring to any particular order or sequence of events. These terms are merely used to distinguish one layer, element, component, region or section from another layer, element, component, region or section. A first layer, element, component, region or section as described herein could be referred to as a second layer, element, component, region or section without departing from the teachings of the present disclosure.

As used herein, the term "or" is used inclusively to refer items in the alternative and in combination.

As used herein, characters such as numerals refer to like elements.

Clause 1. A system for scanning a bone marrow aspirate (BMA) sample, the system comprising: a scanning apparatus for scanning the BMA sample; a processor coupled to the scanning apparatus and a memory and configured to execute instructions which cause the system to: obtain, using the scanning apparatus, scan data of the BMA sample; detect cells in the BMA sample from the scan data; classify the detected cells into a plurality of cell types; and store, in the memory, cell data of the classified cells; and a display configured to present the cell data.

Clause 2. The system of clause 1, wherein processor is configured to detect particles in the BMA sample and detect cells in the BMA sample separate from the detected particles.

Clause 3. The system of clause 2, wherein the particles comprise one or more of fat, or clusters of hematopoietic cells.

Clause 4. The system of clause 1, further comprising an image capture device comprising an effective numerical aperture (NA) of at least 0.8; wherein the detected cells comprise at least 100 cells; and wherein the instructions further comprise instructions for scanning an area of at least 0.5 square cm.

Clause 5. The system of clause 1, wherein classifying the detected cells further comprises identifying a cell lineage linking cells of different cell types and presenting the cell data further comprises presenting the cell lineage.

Clause 6. The system of clause 5, wherein the cell lineage comprises a lineage of the cells from one or more of a myeloid lineage, a lymphoid lineage, or an erythroid lineage.

Clause 7. The system of clause 1, wherein storing the cell data further comprises selecting, from the detected cells, at least relevant cells to store in the cell data.

Clause 8. The system of clause 7, wherein the relevant cells are selected based on a representative distribution of cell types from the detected cells.

Clause 9. The system of clause 7, wherein a number of the relevant cells is between 100-500 cells, 500-1000 cells, 1000-2000 cells, or more than 2000 cells.

Clause 10. The system of clause 1, wherein a scan time for obtaining the scan data is faster than 20 minutes per square cm, 15 minutes per square cm, 10 minutes per square cm, 5 minutes per square cm, or 2 minutes per square cm.

Clause 11. The system of clause 1, wherein the scan data comprises data from at least 95% of an entire area of the BMA sample.

Clause 12. The system of clause 1, wherein the instructions further comprise instructions for: analyzing a preview image corresponding to the BMA sample to locate particles, wherein the preview image has a lower resolution than the scan data; and determining, based on the analysis, a recommended scan area for the BMA sample.

Clause 13. The system of clause 12, wherein the recommended scan area is further determined based on a user input.

Clause 14. The system of clause 13, wherein the user input includes adjusting the recommended scan area to include or exclude a user-defined area.

Clause 15. The system of clause 12, wherein the instructions for obtaining the scan data further comprise instructions for scanning, using the scanning apparatus, the recommended scan area.

Clause 16. The system of clause 1, wherein the instructions further comprise instructions for receiving a user input to modify the cell data.

Clause 17. The system of clause 16, wherein the user input includes at least one of marking a cell, adding a cell, deleting a cell, reclassifying a cell, or adding a notification to a cell.

Clause 18. The system of clause 17, wherein the notification indicates at least one of toxic or abnormal.

Clause 19. The system of clause 1, wherein the scan data includes high-resolution image data of the BMA sample.

Clause 20. The system of clause 19, wherein the high-resolution image data includes image data of the BMA sample scanned with an effective numerical aperture (NA) of at least 0.8.

Clause 21. The system of clause 19, wherein the high-resolution image data includes image data of the BMA sample scanned with an effective numerical aperture (NA) of at least 0.9.

Clause 22. The system of clause 19, wherein the high-resolution image data includes image data of the BMA sample scanned with an effective numerical aperture (NA) of at least 1.0.

Clause 23. The system of clause 1, wherein the scan data includes at least 0.5 square cm of the BMA sample, at least 1 square cm of the BMA sample, at least 1.5 square cm of the BMA sample, or at least 2 square cm of the BMA sample.

Clause 24. The system of clause 1, wherein a number of detected cells is at least one of 500 or more cells, 1000 or more cells, 2000 or more cells, 5000 or more cells, or 10000 or more cells.

Clause 25. The system of clause 1, wherein a number of classified cells is at least one of 500 or more cells, 1000 or more cells, 2000 or more cells, 5000 or more cells, or 10000 or more cells.

Clause 26. The system of clause 1, wherein the processor is configured to classify the cells with a machine learning classifier, the machine learning classifier comprising one or more models selected from the group consisting of a neural network, a convolutional neural network, decision trees, support vector machines, regression analysis, Bayesian networks, and training models.

Clause 27. The system of clause 26, wherein the machine learning classifier is configured to identify a plurality of at least 10 cell types selected from the group consisting of blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, and plasma cells.

Clause 28. The system of clause 1, wherein the scanning apparatus comprises: an illumination assembly configured to illuminate the BMA sample at a plurality of angles; an image capture device configured to collect a plurality of images from the BMA sample illuminated by the illumination assembly at the plurality of angles, the plurality of images comprising a first minimum resolvable distance for features of the cells; and wherein the processor is operatively coupled to the illumination assembly and the image capture device, the processor configured with instructions to generate a computationally reconstructed image from the plurality of images, the computationally reconstructed image comprising a second minimum resolvable distance for the features of the cells, the second minimum resolvable distance less than the first minimum resolvable distance.

Clause 29. The system of clause 1, wherein the processor is configured to a display a cell density heat map.

Clause 30. The system of clause 1, wherein the processor is configured with instructions to identify intact cells and determine an area for further analysis based on locations of the intact cells.

Clause 31. A system for scanning a bone marrow aspirate (BMA) sample comprising: a processor coupled to a memory and configured to execute instructions which cause the system to: receive scan data of the BMA sample; detect particles in the BMA sample from the scan data; detect cells in the BMA sample separate from the detected particles; determine a region of interest (ROI) based on the detected cells; select, based in part on the ROI and the detected cells, a subset of the detected cells for cell data; and store, in the memory, the cell data; and a display configured to present the cell data.

Clause 32. The system of clause 31, wherein to detect the cells further comprises detecting intact cells and the ROI is further determined based on the detected intact cells.

Clause 33. The system of clause 31, wherein to detect the cells further comprises detecting a cell density and the ROI is further determined based on the cell density.

Clause 34. The system of clause 31, wherein the ROI is further determined based on distances between the detected cells and the detected particles.

Clause 35. The system of clause 31, wherein the ROI is further determined based on relevant focal information corresponding to one or more specific locations of the BMA sample.

Clause 36. The system of clause 31, wherein the relevant focal information corresponds to a focal disease with features at the one or more specific locations.

Clause 37. The system of clause 36, wherein the focal disease comprises one or more of lymphoma, plasma cell myeloma, mastocytosis, metastatic carcinoma, storage histiocytosis, crystal storage histiocytosis, or granuloma.

Clause 38. The system of clause 31, wherein detecting the cells further comprises classifying cell types of the cells.

Clause 39. The system of clause 31, wherein the ROI comprises a plurality of ROIs, each of the plurality of ROIs comprising a distribution of cells and wherein a subset of cells is selected from a subset of the plurality of ROIs based on a comparison of a collective distribution of cells from the plurality of ROIs to a total distribution of cells from the ROI.

Clause 40. The system of clause 39, wherein the subset of cells is further selected based on having a cell distribution of cell types representative of a total cell distribution of cell types in the scan data.

Clause 41. The system of clause 39, wherein the subset of cells is further selected based on prioritizing one or more cell types for inclusion into the cell data.

Clause 42. The system of clause 41, wherein the prioritized one or more cell types includes blast cells or plasma cells.

Clause 43. The system of clause 31, wherein presenting the cell data includes presenting a comparison of cell types of a first set of cells to a second set of cells and optionally wherein the comparison comprises a ratio.

Clause 44. The system of clause 43, wherein the first set of cells corresponds to the selected subset of cells.

Clause 45. The system of clause 43, wherein the second set of cells corresponds to a general population of cells from the BMA sample.

Clause 46. The system of clause 31, wherein the processor is further configured to edit the cell data based on a user input.

Clause 47. The system of clause 31, wherein the processor is configured to a display a cell density heat map.

Clause 48. A system for scanning a bone marrow aspirate (BMA) sample comprising: a processor coupled to a memory and configured to execute instructions which cause the system to: receive scan data of the BMA sample; detect cells in the BMA sample; classify the detected cells into one or more cell types; determine, for each of the one or more cell types, a percentage of the cell type with respect to a set of detected cells for cell data; and store, in the memory, the cell data; and a display configured to present the cell data based on a lineage of the one or more cell types.

Clause 49. The system of clause 48, wherein the set of detected cells corresponds to a general population of the detected cells or a chosen subset of the detected cells.

Clause 50. The system of clause 48, wherein the cell data includes a percentage of abnormal cells for each of the one or more cell types.

Clause 51. The system of clause 48, wherein the cell data includes statistical analysis for each of the one or more cell types.

Clause 52. The system of clause 51, wherein the statistical analysis includes at least one of a distribution, an average, or a median.

Clause 53. The system of clause 48, wherein presenting the cell data includes a graphic presentation of the lineage of the one or more cell types.

Clause 54. The system of clause 53, wherein the graphic presentation includes, for each of the one or more cell types, a visual comparison of the percentage of the cell type with respect to the set of detected cells or with respect to a subset of the set of detected cells.

Clause 55. The system of clause 54, wherein the processor is configured with instructions to receive a user input to select the subset of detected cells.

Clause 56. The system of clause 54, wherein the visual comparison includes, for each of the one or more cell types, a shape that is scaled based on the percentage of the cell type.

Clause 57. The system of clause 53, wherein the graphic presentation includes, for each of the one or more cell types, a visual comparison based on a range of values.

Clause 58. The system of clause 53, wherein the graphic presentation includes one or more colors corresponding to one or more cell properties.

Clause 59. The system of clause 48, wherein presenting the cell data includes presenting a cell density heat map.

Clause 60. The system of clause 48, wherein the lineage comprises a lineage of the cells from one or more of a myeloid lineage, a lymphoid lineage, or an erythroid lineage.

Clause 61. A system for scanning a bone marrow aspirate (BMA) sample, the system comprising: a processor coupled to a memory and configured to execute instructions which cause the system to: receive scan data from the BMA sample; detect one or more of particles or cell locations from the scan data; determine an analysis area based on the detected one or more of particles or cell locations; identify cells of one or more cell types from the scan data; analyze the identified cells with statistical analysis; and select, based on the statistical analysis, a subset of the identified cells.

Clause 62. The system of clause 61, wherein determining the analysis area further comprises determining relevant locations based on identifying intact cells.

Clause 63. The system of clause 61, wherein the statistical analysis is based on locations of the identified cells.

Clause 64. The system of clause 61, wherein the statistical analysis is based on comparing cell properties with respect to a general population of the identified cells.

Clause 65. The system of clause 61, further comprising adjusting the statistical analysis based on a user input.

Clause 66. The system of clause 65, wherein the user input includes adjusting the recommended scan area to include or exclude a user-defined area.

Clause 67. The system of clause 61, wherein a second subset of the identified cells comprises randomly selected cells of one or more cell types that were not chosen for the subset of identified cells.

Clause 68. The system of clause 67, wherein the processor is configured with instructions to present data from the second subset to a user to detect bias in the subset.

Clause 69. A method for scanning a bone marrow aspirate (BMA) sample, the method comprising: scanning the BMA sample with a scanning apparatus; obtaining, using the scanning apparatus, scan data of the BMA sample; detecting cells in the BMA sample from the scan data; classifying the detected cells into a plurality of cell types; and storing, in the memory, cell data of the classified cells; and presenting the cell data on a display.

Clause 70. The method of clause 69, wherein particles in the BMA sample are detected and cells in the BMA sample are detected separate from the detected particles.

Clause 71. The method of clause 70, wherein the particles comprise one or more of fat, or clusters of hematopoietic cells.

Clause 72. The method of clause 69, further comprising wherein the sample is scanned with an image capture device comprising an effective numerical aperture (NA) of at least 0.8; wherein the detected cells comprise at least 100 cells; and an area of at least 0.5 square cm is scanned.

Clause 73. The method of clause 69, wherein classifying the detected cells further comprises identifying a cell lineage linking cells of different cell types and presenting the cell data further comprises presenting the cell lineage.

Clause 74. The method of clause 73, wherein the cell lineage comprises a lineage of the cells from one or more of a myeloid lineage, a lymphoid lineage, or an erythroid lineage.

Clause 75. The method of clause 69, wherein storing the cell data further comprises selecting, from the detected cells, at least relevant cells to store in the cell data.

Clause 76. The method of clause 75, wherein the relevant cells are selected based on a representative distribution of cell types from the detected cells.

Clause 77. The method of clause 75, wherein a number of the relevant cells is between 100-500 cells, 500-1000 cells, 1000-2000 cells, or more than 2000 cells.

Clause 78. The method of clause 69, wherein a scan time for obtaining the scan data is faster than 20 minutes per square cm, 15 minutes per square cm, 10 minutes per square cm, 5 minutes per square cm, or 2 minutes per square cm.

Clause 79. The method of clause 69, wherein the scan data comprises data from at least 95% of an entire area of the BMA sample.

Clause 80. The method of clause 69, further comprising: analyzing a preview image corresponding to the BMA sample to locate particles, wherein the preview image has a lower resolution than the scan data; and determining, based on the analysis, a recommended scan area for the BMA sample.

Clause 81. The method of clause 80, wherein the recommended scan area is further determined based on a user input.

Clause 82. The method of clause 81, wherein the user input includes adjusting the recommended scan area to include or exclude a user-defined area.

Clause 83. The method of clause 80, wherein the instructions for obtaining the scan data further comprise instructions for scanning, using the scanning apparatus, the recommended scan area.

Clause 84. The method of clause 69, wherein the instructions further comprise instructions for receiving a user input to modify the cell data.

Clause 85. The method of clause 84, wherein the user input includes at least one of marking a cell, adding a cell, deleting a cell, reclassifying a cell, or adding a notification to a cell.

Clause 86. The method of clause 85, wherein the notification indicates at least one of toxic or abnormal.

Clause 87. The method of clause 69, wherein the scan data includes high-resolution image data of the BMA sample.

Clause 88. The method of clause 87, wherein the high-resolution image data includes image data of the BMA sample scanned with an effective numerical aperture (NA) of at least 0.8.

Clause 89. The method of clause 87, wherein the high-resolution image data includes image data of the BMA sample scanned with an effective numerical aperture (NA) of at least 0.9.

Clause 90. The method of clause 87, wherein the high-resolution image data includes image data of the BMA sample scanned with an effective numerical aperture (NA) of at least 1.0.

Clause 91. The method of clause 69, wherein the scan data includes at least 0.5 square cm of the BMA sample, at least 1 square cm of the BMA sample, at least 1.5 square cm of the BMA sample, or at least 2 square cm of the BMA sample.

Clause 92. The method of clause 69, wherein a number of detected cells is at least one of 500 or more cells, 1000 or more cells, 2000 or more cells, 5000 or more cells, or 10000 or more cells.

Clause 93. The method of clause 69, wherein a number of classified cells is at least one of 500 or more cells, 1000 or more cells, 2000 or more cells, 5000 or more cells, or 10000 or more cells.

Clause 94. The method of clause 69, wherein the cells are classified with a machine learning classifier, the machine learning classifier comprising one or more models selected from the group consisting of a neural network, a convolutional neural network, decision trees, support vector machines, regression analysis, Bayesian networks, and training models.

Clause 95. The method of clause 94, wherein the machine learning classifier is configured to identify a plurality of at least 10 cell types selected from the group consisting of blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, and plasma cells.

Clause 96. The method of clause 69, further comprising: illuminating the BMA sample at a plurality of angles with an illumination assembly; collecting, with an image capture device, a plurality of images from the BMA sample illuminated by the illumination assembly at the plurality of angles, the plurality of images comprising a first minimum resolvable distance for features of the cells; and a computationally reconstructed image is generated from the plurality of images, the computationally reconstructed image comprising a second minimum resolvable distance for the features of the cells, the second minimum resolvable distance less than the first minimum resolvable distance.

Clause 97. The method of clause 69, further comprising displaying a cell density heat map.

Clause 98. The method of clause 69, wherein intact cells are identified and an area for further analysis is determined based on locations of the intact cells.

Clause 99. A method for scanning a bone marrow aspirate (BMA) sample comprising: receiving scan data of the BMA sample; detecting particles in the BMA sample from the scan data; detecting cells in the BMA sample separate from the detected particles; determining a region of interest (ROI) based on the detected cells; selecting, based in part on the ROI and the detected cells, a subset of the detected cells for cell data; and storing, in the memory, the cell data; and presenting the cell data.

Clause 100. The method of clause 99, wherein detecting the cells further comprises detecting intact cells and the ROI is further determined based on the detected intact cells.

Clause 101. The method of clause 99, wherein detecting the cells further comprises detecting a cell density and the ROI is further determined based on the cell density.

Clause 102. The method of clause 99, wherein the ROI is further determined based on distances between the detected cells and the detected particles.

Clause 103. The method of clause 99, wherein the ROI is further determined based on relevant focal information corresponding to one or more specific locations of the BMA sample.

Clause 104. The method of clause 99, wherein the relevant focal information corresponds to a focal disease with features at the one or more specific locations.

Clause 105. The method of clause 104, wherein the focal disease comprises one or more of lymphoma, plasma cell myeloma, mastocytosis, metastatic carcinoma, storage histiocytosis, crystal storage histiocytosis, or granuloma.

Clause 106. The method of clause 99, wherein detecting the cells further comprises classifying cell types of the cells.

Clause 107. The method of clause 99, wherein the ROI comprises a plurality of ROIs, each of the plurality of ROIs comprising a distribution of cells and wherein a subset of cells is selected from a subset of the plurality of ROIs based on a comparison of a collective distribution of cells from the plurality of ROIs to a total distribution of cells from the ROI.

Clause 108. The method of clause 107, wherein the subset of cells is further selected based on having a cell distribution of cell types representative of a total cell distribution of cell types in the scan data.

Clause 109. The method of clause 107, wherein the subset of cells is further selected based on prioritizing one or more cell types for inclusion into the cell data.

Clause 110. The method of clause 109, wherein the prioritized one or more cell types includes blast cells or plasma cells.

Clause 111. The method of clause 99, wherein presenting the cell data includes presenting a comparison of cell types of a first set of cells to a second set of cells and optionally wherein the comparison comprises a ratio.

Clause 112. The method of clause 111, wherein the first set of cells corresponds to the selected subset of cells.

Clause 113. The method of clause 111, wherein the second set of cells corresponds to a general population of cells from the BMA sample.

Clause 114. The method of clause 99, wherein the cell data is edited based on a user input.

Clause 115. The method of clause 99, wherein a display a cell density heat map is displayed.

Clause 116. A method for scanning a bone marrow aspirate (BMA) sample comprising: receiving scan data of the BMA sample; detecting cells in the BMA sample; classifying the detected cells into one or more cell types; determining, for each of the one or more cell types, a percentage of the cell type with respect to a set of detected cells for cell data; and storing, in the memory, the cell data; and presenting the cell data based on a lineage of the one or more cell types.

Clause 117. The method of clause 116, wherein the set of detected cells corresponds to a general population of the detected cells or a chosen subset of the detected cells.

Clause 118. The method of clause 116, wherein the cell data includes a percentage of abnormal cells for each of the one or more cell types.

Clause 119. The method of clause 116, wherein the cell data includes statistical analysis for each of the one or more cell types.

Clause 120. The method of clause 119, wherein the statistical analysis includes at least one of a distribution, an average, or a median.

Clause 121. The method of clause 116, wherein presenting the cell data includes a graphic presentation of the lineage of the one or more cell types.

Clause 122. The method of clause 121, wherein the graphic presentation includes, for each of the one or more cell types, a visual comparison of the percentage of the cell type with respect to the set of detected cells or with respect to a subset of the set of detected cells.

Clause 123. The method of clause 122, wherein a user input is received to select the subset of detected cells.

Clause 124. The method of clause 122, wherein the visual comparison includes, for each of the one or more cell types, a shape that is scaled based on the percentage of the cell type.

Clause 125. The method of clause 121, wherein the graphic presentation includes, for each of the one or more cell types, a visual comparison based on a range of values.

Clause 126. The method of clause 121, wherein the graphic presentation includes one or more colors corresponding to one or more cell properties.

Clause 127. The method of clause 116, wherein presenting the cell data includes presenting a cell density heat map.

Clause 128. The method of clause 116, wherein the lineage comprises a lineage of the cells from one or more of a myeloid lineage, a lymphoid lineage, or an erythroid lineage.

Clause 129. A method for scanning a bone marrow aspirate (BMA) sample, the method comprising: receiving scan data from the BMA sample; detecting one or more of particles or cell locations from the scan data; determining an analysis area based on the detected one or more of particles or cell locations; identifying cells of one or more cell types from the scan data; analyzing the identified cells with statistical analysis; and selecting, based on the statistical analysis, a subset of the identified cells.

Clause 130. The method of clause 129, wherein determining the analysis area further comprises determining relevant locations based on identifying intact cells.

Clause 131. The method of clause 129, wherein the statistical analysis is based on locations of the identified cells.

Clause 132. The method of clause 129, wherein the statistical analysis is based on comparing cell properties with respect to a general population of the identified cells.

Clause 133. The method of clause 129, further comprising adjusting the statistical analysis based on a user input.

Clause 134. The method of clause 129, wherein the user input includes adjusting the recommended scan area to include or exclude a user-defined area.

Clause 135. The method of clause 129, wherein a second subset of the identified cells comprises randomly selected cells of one or more cell types that were not chosen for the subset of identified cells.

Clause 136. The method of clause 135, wherein data from the second subset is presented to a user to detect bias in the subset.

Embodiments of the present disclosure have been shown and described as set forth herein and are provided by way of example only. One of ordinary skill in the art will recognize numerous adaptations, changes, variations and substitutions without departing from the scope of the present disclosure. Several alternatives and combinations of the embodiments disclosed herein may be utilized without departing from the scope of the present disclosure and the inventions disclosed herein. Therefore, the scope of the presently disclosed inventions shall be defined solely by the scope of the appended claims and the equivalents thereof.
THE DISCLOSURE OF THIS APPLICATION ALSO INCLUDES THE FOLLOWING NUMBERED CLAUSES:
1. A system for scanning a bone marrow aspirate (BMA) sample, the system comprising:
   a scanning apparatus for scanning the BMA sample;
      a processor coupled to the scanning apparatus and a memory and configured to execute instructions which cause the system to:
      obtain, using the scanning apparatus, scan data of the BMA sample;
      detect cells in the BMA sample from the scan data;
      classify the detected cells into a plurality of cell types; and
      store, in the memory, cell data of the classified cells; and
   a display configured to present the cell data.
2. The system of clause 1, wherein processor is configured to detect particles in the BMA sample and detect cells in the BMA sample separate from the detected particles.
3. The system of clause 2, wherein the particles comprise one or more of fat, or clusters of hematopoietic cells.
4. The system of clause 1, further comprising
   an image capture device comprising an effective numerical aperture (NA) of at least 0.8;
   wherein the detected cells comprise at least 100 cells; and
   wherein the instructions further comprise instructions for scanning an area of at least 0.5 square cm.
5. The system of clause 1, wherein classifying the detected cells further comprises identifying a cell lineage linking cells of different cell types and presenting the cell data further comprises presenting the cell lineage.
6. The system of clause 5, wherein the cell lineage comprises a lineage of the cells from one or more of a myeloid lineage, a lymphoid lineage, or an erythroid lineage.
7. The system of clause 1, wherein storing the cell data further comprises selecting, from the detected cells, at least relevant cells to store in the cell data.
8. The system of clause 7, wherein the relevant cells are selected based on a representative distribution of cell types from the detected cells.
9. The system of clause 7, wherein a number of the relevant cells is between 100-500 cells, 500-1000 cells, 1000-2000 cells, or more than 2000 cells.
10. The system of clause 1, wherein a scan time for obtaining the scan data is faster than 20 minutes per square cm, 15 minutes per square cm, 10 minutes per square cm, 5 minutes per square cm, or 2 minutes per square cm.
11. The system of clause 1, wherein the scan data comprises data from at least 95% of an entire area of the BMA sample.
12. The system of clause 1, wherein the instructions further comprise instructions for:
   analyzing a preview image corresponding to the BMA sample to locate particles, wherein the preview image has a lower resolution than the scan data; and
   determining, based on the analysis, a recommended scan area for the BMA sample.
13. The system of clause 12, wherein the recommended scan area is further determined based on a user input.
14. The system of clause 13, wherein the user input includes adjusting the recommended scan area to include or exclude a user-defined area.
15. The system of clause 12, wherein the instructions for obtaining the scan data further comprise instructions for scanning, using the scanning apparatus, the recommended scan area.
16. The system of clause 1, wherein the instructions further comprise instructions for receiving a user input to modify the cell data.
17. The system of clause 16, wherein the user input includes at least one of marking a cell, adding a cell, deleting a cell, reclassifying a cell, or adding a notification to a cell.
18. The system of clause 17, wherein the notification indicates at least one of toxic or abnormal.
19. The system of clause 1, wherein the scan data includes high-resolution image data of the BMA sample.
20. The system of clause 19, wherein the high-resolution image data includes image data of the BMA sample scanned with an effective numerical aperture (NA) of at least 0.8.
21. The system of clause 19, wherein the high-resolution image data includes image data of the BMA sample scanned with an effective numerical aperture (NA) of at least 0.9.
22. The system of clause 19, wherein the high-resolution image data includes image data of the BMA sample scanned with an effective numerical aperture (NA) of at least 1.0.
23. The system of clause 1, wherein the scan data includes at least 0.5 square cm of the BMA sample, at least 1 square cm of the BMA sample, at least 1.5 square cm of the BMA sample, or at least 2 square cm of the BMA sample.
24. The system of clause 1, wherein a number of detected cells is at least one of 500 or more cells, 1000 or more cells, 2000 or more cells, 5000 or more cells, or 10000 or more cells.
25. The system of clause 1, wherein a number of classified cells is at least one of 500 or more cells, 1000 or more cells, 2000 or more cells, 5000 or more cells, or 10000 or more cells.
26. The system of clause 1, wherein the processor is configured to classify the cells with a machine learning classifier, the machine learning classifier comprising one or more models selected from the group consisting of a neural network, a convolutional neural network, decision trees, support vector machines, regression analysis, Bayesian networks, and training models.
27. The system of clause 26, wherein the machine learning classifier is configured to identify a plurality of at least 10 cell types selected from the group consisting of blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, and plasma cells.
28. The system of clause 1, wherein the scanning apparatus comprises:
   an illumination assembly configured to illuminate the BMA sample at a plurality of angles;
   an image capture device configured to collect a plurality of images from the BMA sample illuminated by the illumination assembly at the plurality of angles, the plurality of images comprising a first minimum resolvable distance for features of the cells; and
   wherein the processor is operatively coupled to the illumination assembly and the image capture device, the processor configured with instructions to generate a computationally reconstructed image from the plurality of images, the computationally reconstructed image comprising a second minimum resolvable distance for the features of the cells, the second minimum resolvable distance less than the first minimum resolvable distance.
29. The system of clause 1, wherein the processor is configured to a display a cell density heat map.
30. The system of clause 1, wherein the processor is configured with instructions to identify intact cells and determine an area for further analysis based on locations of the intact cells.
31. A system for scanning a bone marrow aspirate (BMA) sample comprising:
   a processor coupled to a memory and configured to execute instructions which cause the system to:
      receive scan data of the BMA sample;
      detect particles in the BMA sample from the scan data;
      detect cells in the BMA sample separate from the detected particles;
      determine a region of interest (ROI) based on the detected cells;
      select, based in part on the **ROI** and the detected cells, a subset of the detected cells for cell data; and
      store, in the memory, the cell data; and
   a display configured to present the cell data.
32. The system of clause 31, wherein to detect the cells further comprises detecting intact cells and the **ROI** is further determined based on the detected intact cells.
33. The system of clause 31, wherein to detect the cells further comprises detecting a cell density and the **ROI** is further determined based on the cell density.
34. The system of clause 31, wherein the **ROI** is further determined based on distances between the detected cells and the detected particles.
35. The system of clause 31, wherein the **ROI** is further determined based on relevant focal information corresponding to one or more specific locations of the BMA sample.
36. The system of clause 31, wherein the relevant focal information corresponds to a focal disease with features at the one or more specific locations.
37. The system of clause 36, wherein the focal disease comprises one or more of lymphoma, plasma cell myeloma, mastocytosis, metastatic carcinoma, storage histiocytosis, crystal storage histiocytosis, or granuloma.
38. The system of clause 31, wherein detecting the cells further comprises classifying cell types of the cells.
39. The system of clause 31, wherein the ROI comprises a plurality of ROIs, each of the plurality of ROIs comprising a distribution of cells and wherein a subset of cells is selected from a subset of the plurality of ROIs based on a comparison of a collective distribution of cells from the plurality of ROIs to a total distribution of cells from the ROI.
40. The system of clause 39, wherein the subset of cells is further selected based on having a cell distribution of cell types representative of a total cell distribution of cell types in the scan data.
41. The system of clause 39, wherein the subset of cells is further selected based on prioritizing one or more cell types for inclusion into the cell data.
42. The system of clause 41, wherein the prioritized one or more cell types includes blast cells or plasma cells.
43. The system of clause 31, wherein presenting the cell data includes presenting a comparison of cell types of a first set of cells to a second set of cells and optionally wherein the comparison comprises a ratio.
44. The system of clause 43, wherein the first set of cells corresponds to the selected subset of cells.
45. The system of clause 43, wherein the second set of cells corresponds to a general population of cells from the BMA sample.
46. The system of clause 31, wherein the processor is further configured to edit the cell data based on a user input.
47. The system of clause 31, wherein the processor is configured to a display a cell density heat map.
48. A system for scanning a bone marrow aspirate (BMA) sample comprising:
   a processor coupled to a memory and configured to execute instructions which cause the system to:
      receive scan data of the BMA sample;
      detect cells in the BMA sample;
      classify the detected cells into one or more cell types;
      determine, for each of the one or more cell types, a percentage of the cell type with respect to a set of detected cells for cell data; and
      store, in the memory, the cell data; and
   a display configured to present the cell data based on a lineage of the one or more cell types.
49. The system of clause 48, wherein the set of detected cells corresponds to a general population of the detected cells or a chosen subset of the detected cells.
50. The system of clause 48, wherein the cell data includes a percentage of abnormal cells for each of the one or more cell types.
51. The system of clause 48, wherein the cell data includes statistical analysis for each of the one or more cell types.
52. The system of clause 51, wherein the statistical analysis includes at least one of a distribution, an average, or a median.
53. The system of clause 48, wherein presenting the cell data includes a graphic presentation of the lineage of the one or more cell types.
54. The system of clause 53, wherein the graphic presentation includes, for each of the one or more cell types, a visual comparison of the percentage of the cell type with respect to the set of detected cells or with respect to a subset of the set of detected cells.
55. The system of clause 54, wherein the processor is configured with instructions to receive a user input to select the subset of detected cells.
56. The system of clause 54, wherein the visual comparison includes, for each of the one or more cell types, a shape that is scaled based on the percentage of the cell type.
57. The system of clause 53, wherein the graphic presentation includes, for each of the one or more cell types, a visual comparison based on a range of values.
58. The system of clause 53, wherein the graphic presentation includes one or more colors corresponding to one or more cell properties.
59. The system of clause 48, wherein presenting the cell data includes presenting a cell density heat map.
60. The system of clause 48, wherein the lineage comprises a lineage of the cells from one or more of a myeloid lineage, a lymphoid lineage, or an erythroid lineage.
61. A system for scanning a bone marrow aspirate (BMA) sample, the system comprising:
   a processor coupled to a memory and configured to execute instructions which cause the system to:
   receive scan data from the BMA sample;
   detect one or more of particles or cell locations from the scan data;
   determine an analysis area based on the detected one or more of particles or cell locations;
   identify cells of one or more cell types from the scan data;
   analyze the identified cells with statistical analysis; and
   select, based on the statistical analysis, a subset of the identified cells.
62. The system of clause 61, wherein determining the analysis area further comprises determining relevant locations based on identifying intact cells.
63. The system of clause 61, wherein the statistical analysis is based on locations of the identified cells.
64. The system of clause 61, wherein the statistical analysis is based on comparing cell properties with respect to a general population of the identified cells.
65. The system of clause 61, further comprising adjusting the statistical analysis based on a user input.
66. The system of clause 65, wherein the user input includes adjusting the recommended scan area to include or exclude a user-defined area.
67. The system of clause 61, wherein a second subset of the identified cells comprises randomly selected cells of one or more cell types that were not chosen for the subset of identified cells.
68. The system of clause 67, wherein the processor is configured with instructions to present data from the second subset to a user to detect bias in the subset.

## Claims

1. A system for scanning a bone marrow aspirate (BMA) sample, the system comprising:
a scanning apparatus for scanning the BMA sample;
a processor coupled to the scanning apparatus and a memory and configured to execute instructions which cause the system to:
obtain, using the scanning apparatus, scan data of the BMA sample;
detect cells in the BMA sample from the scan data;
classify the detected cells into a plurality of cell types; and
store, in the memory, cell data of the classified cells; and
a display configured to present the cell data.

2. The system of claim 1, wherein processor is configured to detect particles in the BMA sample and detect cells in the BMA sample separate from the detected particles.

3. The system of claim 2, wherein the particles comprise one or more of fat, or clusters of hematopoietic cells.

4. The system of claim 1, further comprising
an image capture device comprising an effective numerical aperture (NA) of at least 0.8;
wherein the detected cells comprise at least 100 cells; and
wherein the instructions further comprise instructions for scanning an area of at least 0.5 square cm.

5. The system of claim 1, wherein classifying the detected cells further comprises identifying a cell lineage linking cells of different cell types and presenting the cell data further comprises presenting the cell lineage.

6. The system of claim 5, wherein the cell lineage comprises a lineage of the cells from one or more of a myeloid lineage, a lymphoid lineage, or an erythroid lineage.

7. The system of claim 1, wherein storing the cell data further comprises selecting, from the detected cells, at least relevant cells to store in the cell data.

8. The system of claim 7, wherein a number of the relevant cells is between 100-500 cells, 500-1000 cells, 1000-2000 cells, or more than 2000 cells.

9. The system of claim 1, wherein a scan time for obtaining the scan data is faster than 20 minutes per square cm, 15 minutes per square cm, 10 minutes per square cm, 5 minutes per square cm, or 2 minutes per square cm.

10. The system of claim 1, wherein the scan data comprises data from at least 95% of an entire area of the BMA sample.

11. The system of claim 1, wherein the instructions further comprise instructions for:
analyzing a preview image corresponding to the BMA sample to locate particles, wherein the preview image has a lower resolution than the scan data; and
determining, based on the analysis, a recommended scan area for the BMA sample.

12. The system of claim 11, wherein the recommended scan area is further determined based on a user input, wherein the user input includes adjusting the recommended scan area to include or exclude a user-defined area.

13. The system of claim 1, wherein the processor is configured to classify the cells with a machine learning classifier, the machine learning classifier comprising one or more models selected from the group consisting of a neural network, a convolutional neural network, decision trees, support vector machines, regression analysis, Bayesian networks, and training models.

14. The system of claim 13, wherein the machine learning classifier is configured to identify a plurality of at least 10 cell types selected from the group consisting of blasts, immature eosinophils, eosinophils, immature basophils, basophils, promelocytes, myelocytes, metamyelocytes, bands, neutrophils, monoblasts, monocytes, macrophages, megakaryoblasts, megakaryocytes, erythroblasts, megaloblasts, normoblasts, lymphocytes, and plasma cells.
